# EUROPEAN PATENT APPLICATION

(11) **EP 3 733 837 A1**
(43) Date of publication of application: **04.11.2020**
(21) Application number: 18894959.8
(22) Date of filing: 27.12.2018
(51) Int. Cl.: C12N 5/071, C12N 5/0735

(54) **CELL AGGREGATION PROMOTING AGENT**

(30) Priority: 28.12.2017 JP 2017254688
(71) Applicant: Kaneka Corporation, Osaka-shi, Osaka 530-8288 (JP)
(72) Inventor: IBUKI Masato, Kobe-shi, Hyogo 650-0047 (JP)
(74) Representative: Hoffmann Eitle
(86) International application number: PCT/JP2018/048314
(87) International publication number: WO 2019/131942

(57) **Abstract**

The present invention is directed to providing a means for appropriately controlling the size of cell aggregates without relying on mechanical/physical means. Specifically, the present invention relates to a cell aggregation promoter for use in suspension culture of cells, comprising an SRF inhibitor. The present invention also relates to a method for producing cell aggregates, comprising a step of culturing cells in suspension in a culture medium comprising an SRF inhibitor.

## Description

### Technical Field

The present invention relates to cell aggregation promoters. The present invention also relates to methods for producing cell aggregates and cell aggregates produced thereby. The present invention also relates to cell culture compositions.

### Background Art

Recent research on human pluripotent stem cells (e.g., human ES cells and human iPS cells) has increasingly made regenerative medicine come in reality. These cells possess an ability to proliferate infinitely and an ability to differentiate into various types of cells. Thus, regenerative medicine using the pluripotent stem cells should radically change therapeutic interventions against, for example, refractory diseases and lifestyle-related diseases. It has already been possible that the pluripotent stem cells can be induced and differentiated *in vitro* into various types of cells including neurons, cardiomyocytes, blood cells, and retinal cells.

One of objectives directed toward practical use of regenerative medicine in which pluripotent stem cells are used to regenerate a variety of organs involves how a large number of cells necessary for regeneration of organs can be produced efficiently. For example, the regeneration of a liver requires about 2 × 10¹¹ cells. A substrate plate with an area of 10⁶ cm² or more is needed so as to culture the above number of cells using adherent culture on a flat substrate plate. This means that about 20,000 common 10-cm dishes are needed. Because the number of cells to be obtained using adherent culture on a surface of the substrate plate depends on the surface area of the culture plate, it is difficult to scale up the culture. Accordingly, it is hard to provide an enough number of cells to make regenerative medicine available.

Here, it is easy to scale up suspension culture in which cells are cultured in suspension in a liquid culture medium. Hence, the suspension culture should be fit for mass production of cells.

For example, Non-Patent Literature 1 discloses a process for producing spheroids with a uniform size, the process comprising: using a spinner flask as cell cultureware for suspension culture; and culturing human pluripotent stem cells in suspension while strongly stirring a liquid culture medium.

Non-Patent Literature 2 discloses a process for producing spheroids with a uniform size in each micro-well, the process comprising using a substrate plate on which small micro-wells are formed.

Non-Patent Literature 3 discloses a culturing method comprising: using a culture medium the viscosity and specific gravity of which is adjusted; keeping pluripotent stem cells in suspension; and reducing a collision between the cells.

Patent Literature 1 discloses a technology in which cells are cultured while being subjected to rotary shaking culture in a liquid culture medium, so that cell aggregates are produced.

Patent Literature 2 discloses a method in which pluripotent stem cells are cultured in suspension until the average diameter of cell aggregates reaches about 200 µm or more and 300 µm or less.

Patent Literature 3 discloses a technique for suppressing cell aggregation by culturing cells in suspension in a culture medium containing lysophospholipids such as lysophosphatidic acid (LPA) and sphingosine-1-phosphate (SIP).

### Citation List

### Patent Literature

Patent Literature 1: JP Patent Publication (Kokai) No. 2003-304866A
Patent Literature 2: WO2013/077423A
Patent Literature 3: WO2016/121737A

### Non-Patent Literature

Non-Patent Literature 1: Olmer R. et al., Tissue Engineering: Part C, Volume 18 (10): 772-784 (2012)
Non-Patent Literature 2: Ungrin MD et al., PLoS ONE, 2008, 3(2), e1565
Non-Patent Literature 3: Otsuji GT et al., Stem Cell Reports, Volume 2: 734-745 (2014)

### Summary of Invention

### Technical Problem

The present inventors have found that non-specific adsorption of membrane proteins and cell membranes between cells, and adhesion between cells mediated by cadherin on the cell surface are important mechanisms in culturing adhesive cells such as pluripotent stem cells in suspension. That is, a challenge that needs to be addressed in the technique of suspension culture is to produce cell aggregates with an appropriate size without damaging the bindings of membrane proteins of the cells, cadherins on the cell surface, or the like. However, the techniques of suspension culture disclosed in Non-Patent Literatures 1-3 and Patent Literatures 1 or 2 had the following problems:

The process of Non-Patent Literature 1 likely causes cells to die due to shear stress, which is a defect of the process.

In the process of Non-Patent Literature 2, it is difficult to scale up a culture and to change a culture medium.

In the method of Non-Patent Literature 3, because of less movement of a culture medium during culture, oxygen and nutritional components are less likely to be supplied to cell aggregates.

Patent Literature 1 fails to disclose a means for controlling the size of cell aggregates to an appropriate size.

Patent Literature 2 discloses adding, to a culture medium, a water-soluble polymer as a means for preventing adhesion between cell aggregates, so that the viscosity increases. This causes the same defect as in the case of Non-Patent Literature 3, in which oxygen and nutritional components are less likely to be supplied to cell aggregates.

In order to solve the above problems, the present inventors have developed a technique for suspension culture that can suppress cell aggregation by culturing cells in suspension in a culture medium containing lysophospholipids in Patent Literature 3 and produce aggregates with an appropriate size without damaging the cells.

However, Patent Literature 3 discloses only lysophospholipids as components that suppress cell aggregation. The present inventors have contemplated that the culture medium in suspension culture provided with components that suppress or promote cell aggregation in addition to lysophospholipids would allow the size of cell aggregates to be appropriately controlled without relying on mechanical/physical means.

### Solution to Problem

As a result of extensive researches, the present inventors have found that SRF inhibitors exhibit an action of promoting cell aggregation by being present in a culture medium in suspension culture. Based on this finding, the present inventors have completed the present invention.
(1) A cell aggregation promoter for use in suspension culture of cells, comprising an SRF inhibitor.
(2) The cell aggregation promoter according to item (1), wherein a concentration of the SRF inhibitor is 9.0 µg/mL or more and 10.0 mg/mL or less.
(3) The cell aggregation promoter according to item (1) or (2), further comprising a ROCK inhibitor.
(4) The cell aggregation promoter according to any one of items (1) to (3), wherein the SRF inhibitor is CCG-1423.
(5) The cell aggregation promoter according to any one of items (1) to (4), wherein the cells are stem cells.
(6) A method for producing cell aggregates, comprising a step of culturing cells in suspension in a culture medium comprising an SRF inhibitor.
(7) The method according to item (6), wherein a concentration of the SRF inhibitor in the culture medium is 4.5 ng/mL or more and 4.6 mg/mL or less.
(8) The method according to item (6) or (7), wherein the culture medium further comprises a ROCK inhibitor.
(9) The method according to any one of items (6) to (8), wherein the SRF inhibitor is CCG-1423.
(10) The method according to any one of items (6) to (9), wherein the cells are stem cells.
(11) A cell aggregate obtained by the method according to any one of items (6) to (10).
(12) A cell culture composition comprising cells, a culture medium, and an SRF inhibitor.
(13) The cell culture composition according to (12) wherein a concentration of the SRF inhibitor is 4.5 ng/mL or more and 4.6 mg/mL or less.
(14) The cell culture composition according to item (12) or (13), further comprising a ROCK inhibitor.
(15) The cell culture composition according to any one of items (12) to (14), wherein the SRF inhibitor is CCG-1423.
(16) The cell culture composition according to any one of items (12) to (15), wherein the cells are stem cells.
(17) The cell culture composition according to any one of items (12) to (16), wherein the cells are in a form of cell aggregates.
(18) A method for promoting a cell aggregation, comprising a step of culturing cells in suspension in a culture medium comprising an SRF inhibitor.
(19) The method according to item (18), wherein a concentration of the SRF inhibitor in the culture medium is 4.5 ng/mL or more and 4.6 mg/mL or less.
(20) The method according to item (18) or (19), wherein the culture medium further comprises a ROCK inhibitor.
(21) The method according to any one of items (18) to (20), wherein the SRF inhibitor is CCG-1423.
(22) The method according to any one of items (18) to (21), wherein the cells are stem cells.
(23) A cell culture medium comprising a culture medium and an SRF inhibitor.
(24) The cell culture medium according to item (23), wherein a concentration of the SRF inhibitor is 4.5 ng/mL or more and 4.6 mg/mL or less.
(25) The cell culture medium according to item (23) or (24), further comprising a ROCK inhibitor.
(26) The cell culture medium according to any one of items (23) to (25), further comprising a growth factor.
(27) A cell culture medium according to any one of items (23) to (26) for use in culture of stem cells.
(28) A cell culture medium according to any one of items (23) to (27) for producing cell aggregates.
(29) The method according to any one of items (6) to (10), the cell aggregate according to item (11), the cell culture composition according to item (17), or the cell culture medium according to item (28), wherein a size of the widest portion in 70% or more (by weight) of the cell aggregates is 500 µm or less, preferably 300 µm or less.
(30) The method according to any one of items (6) to (10) and item (29), the cell aggregate according item (11) or (29), the cell culture composition according to item (17) or (29), or the cell culture medium according to item (28) or (29), wherein a size of the widest portion in 70% or more (by weight) of the cell aggregates is 40 µm or more, preferably 100 µm or more.

The text of specification includes disclosure of JP Patent Application No. 2017-254688, of which the present application claims priority.

### Advantageous Effects of Invention

One embodiment of the cell aggregation promoter of the present invention can be mixed in a culture medium to promote cell aggregation during suspension culture.

According to one embodiment of the method for producing a cell aggregate of the present invention, cell aggregates can be produced in high yields.

One embodiment of the cell aggregate of the present invention has an appropriate size and high viable cell ratio.

One embodiment of the cell culture composition of the present invention can be used to produce cell aggregates in high yields.

### Brief Description of the Drawings

[Figure 1] Figure 1 shows observation images by phase contrast microscopy at Day 1 of culture when human iPS cells were cultured in suspension in a CCG-1423 added or non-added culture medium.
[Figure 2] Figure 2 shows the measurement result of the number of dead cells at Day 1 of culture when human iPS cells were cultured in suspension in a CCG-1423 added or non-added culture medium.
[Figure 3] Figure 3 shows the observation images by phase contrast microscopy from Day 1 to Day 5 of culture when human iPS cells were cultured in suspension in a CCG-1423 added or non-added culture medium and, after cell aggregates were produced, continuously cultured in suspension.
[Figure 4] Figure 4 shows the measurement result of glucose consumption from Day 1 to Day 5 of culture when human iPS cells were cultured in suspension in a CCG-1423 added or non-added culture medium and, after cell aggregates were produced, continuously cultured in suspension.
[Figure 5] Figure 5 shows the measurement result of cell yields at Day 5 of culture when human iPS cells were cultured in suspension in a CCG-1423 added or non-added culture medium and, after cell aggregates were produced, continuously cultured in suspension.
[Figure 6] Figure 6 shows the measurement result of the percentage of human iPS cells at Day 5 of culture positive for undifferentiation markers (SOX2, OCT4, and Nanog) when the cells were cultured in suspension in a CCG-1423 added culture medium and, after cell aggregates were produced, continuously cultured in suspension.
[Figure 7] Figure 7 is a graph showing the distribution of diameters of cell aggregates at Day 1 of culture when human iPS cells were cultured in suspension in a culture medium containing CCG-1423 (Day 1 of culture) and cell aggregates were produced.

### Description of Embodiments

Hereinafter, preferable embodiments of the present invention will be described in detail.

### <1. Cells>

Aggregate-forming cells in the present invention may be cells that are adherent (adherent cells). Examples of the adherent cells may include: animal-derived cells; preferably mammalian-derived cells; more preferably biological tissue-derived cells and cells derived from the biological tissue-derived cells; particularly preferably epithelial tissue-derived cells and cells derived from the epithelial tissue-derived cells, connective tissue-derived cells and cells derived from the connective tissue-derived cells, muscular tissue-derived cells and cells derived from the muscular tissue-derived cells, or nervous tissue-derived cells and cells derived from the nervous tissue-derived cells; further more preferably animal-derived stem cells and cells differentiated from the animal-derived stem cells; still more preferably animal-derived pluripotent stem cells and cells differentiated from the animal-derived pluripotent stem cells; still more preferably mammalian-derived pluripotent stem cells and cells differentiated from the mammalian-derived pluripotent stem cells; and most preferably human-derived pluripotent stem cells and cells differentiated from the human-derived pluripotent stem cells.

As used herein, the "stem cell" is a cell that is capable of differentiation into another cell and has a self-replicating activity. Among the "stem cells", a cell that has a multipotency (pluripotency) capable of differentiating into all types of cells constituting a living body and that can continue to proliferate infinitely while maintaining its pluripotency during *in vitro* culture under suitable conditions is referred to as a "pluripotent stem cell". Specific examples of the pluripotent stem cells include, but are not limited to, embryonic stem cells (ES cells), EG cells, which are pluripotent stem cells derived from fetal primordial germ cells, (Shamblott M. J. et al., Proc. Natl. Acad. Sci. USA. (1998) 95, p.13726-13731), GS cells, which are testis-derived pluripotent stem cells, (Conrad S., Nature (2008) 456, p.344-349), and iPS cells (induced pluripotent stem cells), which are somatic cell-derived induced pluripotent stem cells. Regarding the pluripotent stem cells used in the present invention, particularly preferred are ES cells or iPS cells. ES cells are pluripotent stem cells derived from an early embryo called a blastocyst. iPS cells are cultured cells produced by introducing reprogramming factors into a somatic cell, so that the somatic cell is reprogrammed into an undifferentiated state and is given pluripotency. Examples of the reprogramming factors that can be used include OCT3/4, KLF4, SOX2, and c-Myc (Takahashi K, et al. Cell. 2007; 131:861-72). For example, OCT3/4, SOX2, LIN28, and Nanog may be used (Yu J, et al. Science. 2007; 318:1917-20). Examples of how to introduce these factors into a cell include, but are not particularly limited to, a plasmid-mediated gene transfer, synthetic RNA introduction, and a direct injection of a protein(s). In addition, it may be possible to use iPS cells that are created using, for example, microRNA, RNA, and/or a low-molecular-weight compound. As the pluripotent stem cells (including the ES cells, iPS cells, etc.), commercially available products or cells obtained from a third party may be used or freshly prepared ones may be used. Examples of iPS cell lines that can be used include 253G1, 201B6, 201B7, 409B2, 454E2, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC 38-2, MSC-iPSC1, and BJ-iPSC1. Examples of ES cell lines that can be used include KhES-1, KhES-2, KhES-3, KhES-4, KhES-5, SEES-1, SEES-2, SEES-3, SEES-4, SEES-5, SEES-6, SEES-7, HUES8, CyT49, H1, H9, HS-181, and RPChiPS771-2. Also, freshly prepared clinical-grade iPS or ES cells may be used. Examples of the origin of cells when iPS cells are created include, but are not particularly limited to, fibroblasts and lymphocytes.

The types of cells in the present invention are not particularly limited as long as they are cells capable of adhering to plastics or cells by extracellular matrices, cadherins, or the like. Examples thereof include pluripotent stem cells described above (e.g., induced pluripotent stem cells (iPS cells), embryonic stem cells (ES cells), GS cells that are pluripotent stem cells derived from testis, EG cells derived from fetal primordial germ cells, or Muse cells derived from bone marrow or the like), somatic stem cells (e.g., mesenchymal stem cells derived from bone marrow, adipose tissue, dental marrow, placenta, ovum, umbilical cord blood, amniotic membrane, chorionic membrane or the like, or neural stem cells), neuronal cells, cardiomyocytes, cardiomyocardial progenitor cells, hepatocytes, hepatic progenitor cells, α cells, β cells, fibroblasts, chondrocytes, corneal cells, vascular endothelial cells, vascular endothelial progenitor cells, and peripheral cells. The cells may be in a form into which a gene is introduced or a form in which a gene of interest on the genome is knockdowned.

Cells used in the present invention may be originated from any animal. Examples of the origin may include: mammals such as rodents (e.g., a mouse, rat, hamster), primates (e.g., a human, gorilla, chimpanzee), and domestic animals and pets (e.g., a dog, cat, rabbit, cow, horse, sheep, goat). Particularly preferred are human cells.

In the present invention, cells isolated after undergoing adherent or suspension culture may be used. Here, the term "isolated cells" means cells obtained by detaching and dispersing a cell population composed of a plurality of cells adhering to one another. The isolation involves the step of detaching and dispersing cells adhering to, for example, cultureware and/or a culture support or a cell population, in which cells adhere to one another, to give single cells. The cell population to be isolated may be in suspension in a liquid culture medium. Preferable examples of the isolation procedure may include, but are not particularly limited to, a procedure using a detachment agent (e.g., a cell detachment enzyme such as trypsin or collagenase), a chelating agent (e.g., EDTA (ethylene diamine tetraacetic acid)), or a mixture of the detachment agent and the chelating agent. Examples of the detachment agent include, but are not particularly limited to, trypsin, Accutase (a registered trade mark), TrypLE™ Express Enzyme (Life Technologies Japan Ltd.), TrypLE™ Select Enzyme (Life Technologies Japan Ltd.), "Dispase" (a registered trade mark), and collagenase. The cells that have been isolated, frozen, and stored after the isolation procedure may be preferably used in the present invention.

### <2. Cell Aggregates>

A cell aggregate refers to what is called a spheroid that is a clustered cell population formed while a plurality of cells aggregate three-dimensionally. Cell aggregates typically have a substantially spherical shape.

As used herein, the "cell aggregation" refers to assembling a plurality of cells in three dimensions to form aggregates. The aggregation used herein includes both aggregation of different cells and aggregation of identical cells. The aggregation of identical cells includes an aggregation in which an aggregate is formed by the proliferation of one cell. The mechanism of cell aggregation is not limited, and examples thereof include non-specific adsorption of membrane proteins and cell membranes between cells, and adhesion between cells mediated by cadherin on the cell surface.

In the present invention, cells that constitute a cell aggregate are not particularly limited as long as they are one or more type of cells described above. For example, a cell aggregate composed of pluripotent stem cells such as human pluripotent stem cells or human embryonic stem cells includes cells expressing a pluripotent stem cell marker and/or positive for a pluripotent stem cell marker. Examples of the pluripotent stem cell maker include alkaline phosphatase, NANOG, OCT4, SOX2, TRA-1-60, c-Myc, KLF4, LIN28, SSEA-4, and SSEA-1.

The pluripotent stem cell marker can be detected by any detection method in the art. Examples of the method for detecting expression markers include, but are not limited to, flow cytometry. In flow cytometry using a fluorescently labeled antibody, when cells emitting greater fluorescence compared to negative control (isotype control) are detected, the cells are determined to be "positive" for the marker. The percentage of cells positive for fluorescently labeled antibodies analyzed by flow cytometry is sometimes referred to as a positive ratio. As the fluorescently labeled antibodies, any antibody known in the art can be used, and examples of the antibodies include, but are not limited to, antibodies labeled with fluorescein isothiocyanate (FITC), phycoerythrin (PE), allophycocyanin (APC), or the like.

When cells that constitute the cell aggregate are pluripotent stem cells, the percentage (ratio) of cells that express pluripotent stem cell markers and/or are positive for pluripotent stem cell markers may be, for example, 80% or more, 90% or more, 91% or more, 92% or more, 93% or more, 94% or more, 95% or more, 96% or more, 97% or more, 98% or more, or 99% or more, and 100% or less. The cell aggregates in which the percentage of cells that express pluripotent stem cell markers and/or are positive for pluripotent stem cell markers is within the above-mentioned range are a more undifferentiated and more homogeneous cell population. Note that pluripotent stem cell markers are synonymous with undifferentiation markers, and both can be used interchangeably.

The size of the cell aggregate produced by one or more embodiments of the present invention is not particularly limited, and when observed under a microscope, the upper limit of the size of the widest portion in an observation image is, for example, 1000 µm or less, 900 µm or less, 800 µm or less, 700 µm or less, 600 µm or less, 500 µm or less, 400 µm or less, 300 µm or less, or 200 µm or less. The lower limit is, for example, 40 µm or more, 50 µm or more, 60 µm or more, 70 µm or more, 80 µm or more, 90 µm or more, or 100 µm or more. As a side note, one human iPS cell is about 10 µm in size. The cell aggregates with such a size range have a preferable cell proliferation environment for because oxygen and nutritional components are easily supplied to their inner cells.

It is preferable in a cell aggregate population produced by one or more embodiments of the present invention that, when measured by weight, for example, 10% or higher, 20% or higher, 30% or higher, 40% or higher, 50% or higher, 60% or higher, 70% or higher, 80% or higher, or 90% or higher of cell aggregates constituting the cell aggregate population have a size within the above-mentioned range. The cell aggregate population containing 20% or higher of cell aggregates having a size within the above-mentioned range has a preferable cell proliferation environment because oxygen and nutritional components are easily supplied to their inner cells in individual cell aggregates.

It is preferable in a cell aggregate population produced by one or more embodiments of the present invention that the percentage of viable cells (viability) in cells constituting the cell aggregate population is, for example, 50% or more, 60% or more, 70% or more, 80% or more, or 90% or more. The cell aggregates having a viability within the above-mentioned range easily maintain the aggregate state and are in a preferred state for cell proliferation.

### <3. Culture Medium>

The culture medium used in the present invention can be prepared by using any culture medium for culturing an animal cell as a basal medium and appropriately adding an SRF inhibitor or an SRF inhibitor and a ROCK inhibitor, or a cell aggregation promoter comprising an SRF inhibitor or an SRF inhibitor and a ROCK inhibitor, and other components as needed to the basal medium. It is preferable that the culture medium used in the present invention is a culture medium suitable for suspension culture of cells, and typically a liquid medium.

Examples of the basal medium that can be used include, but are not particularly limited to, BME medium, BGJb medium, CMRL1066 medium, Glasgow MEM medium, Improved MEM Zinc Option medium, IMDM medium (Iscove's Modified Dulbecco's Medium), Medium 199 medium, Eagle MEM medium, αMEM medium, DMEM medium (Dulbecco's Modified Eagle's Medium), Ham's F10 medium, Ham's F12 medium, RPMI 1640 medium, Fischer's medium, and a mixed medium thereof (e.g., DMEM/F12 medium (Dulbecco's Modified Eagle's Medium/Nutrient Mixture F-12 Ham)). The DMEM/F12 medium may be used, in particular, by mixing DMEM medium and Ham's F12 medium in a weight ratio of preferably from 60/40 or more to 40/60 or less, more preferably from 55/45 or more to 45/55 or less, and most preferably in a weight ratio of 50/50.

The culture medium used in the present invention is preferably a medium containing no serum, namely a serum-free medium or a medium containing serum replacement. Examples of the serum replacement include KnockOut™ Serum Replacement (KSR) (Gibco).

The culture medium used in the present invention preferably contains at least one selected from L-ascorbic acid, insulin, transferrin, selenium and sodium bicarbonate, and more preferably contains all of these. The L-ascorbic acid, insulin, transferrin, selenium, and sodium bicarbonate may be added to the medium in the form of, for example, a solution, derivative, salt, or mixed reagent. For example, L-ascorbic acid may be added to the medium in the form of a derivative such as magnesium-ascorbyl-2-phosphate. Selenium may be added to the medium in the form of a selenite (e.g., sodium selenite). The insulin and transferrin may be natural ones isolated from a tissue or serum of an animal (e.g., preferably a human, mouse, rat, cow, horse, goat). They may be genetically engineered recombinant proteins. The insulin, transferrin, and selenium may be added to the medium in the form of a reagent ITS (insulin-transferrin-selenium). The ITS is a cell growth-promoting additive containing insulin, transferrin, and sodium selenite.

A commercially available culture medium containing at least one selected from L-ascorbic acid, insulin, transferrin, selenium, and sodium bicarbonate may be used. Examples of a commercially available culture medium supplemented with insulin and transferrin may include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE™ (BioWhittaker, Inc.), UltraDOMA™ (BioWhittaker, Inc.), UltraCHO™ (BioWhittaker, Inc.), and UltraMDCK™ (BioWhittaker, Inc.). For, example, STEMPRO (a registered trade mark), hESC SFM (Life Technologies Japan Ltd.), mTeSR1 (Veritas, Ltd.), or TeSR2 (Veritas, Ltd.) may be preferably used. In addition, it is preferable to use a culture medium used for culturing human iPS cells and/or human ES cells.

The culture medium used in the present invention preferably contains at least one growth factor. The liquid culture medium preferably contains at least one growth factor, which is not limited to the following, selected from the group consisting of FGF2 (basic fibroblast growth factor-2), TGF-β1 (transforming growth factor-β1), Activin A, IGF-1, MCP-1, IL-6, PAI, PEDF, IGFBP-2, LIF, and IGFBP-7. Particularly preferred growth factor is FGF2 and/or TGF-β1.

The most preferable culture medium used in the present invention is a serum-free medium containing, in addition to the SRF inhibitor and the ROCK inhibitor described below, components: L-ascorbic acid, insulin, transferrin, selenium, and sodium bicarbonate as well as at least one growth factor. Particularly preferred is a serum-free DMEM/F12 medium containing L-ascorbic acid, insulin, transferrin, selenium, and sodium bicarbonate as well as at least one growth factor (preferably, FGF2 and TGF-β1). Examples of such a culture medium that can be preferably used include Essential 8™ culture medium (Life Technologies Japan Ltd.) supplemented with an SRF inhibitor or an SRF inhibitor and a ROCK inhibitor. The Essential 8™ medium may be prepared by mixing DMEM/F-12 (HAM) (1:1), which is a DMEM/F12 medium marketed by Life Technologies Japan Ltd., and Essential 8™ supplement (containing L-ascorbic acid, insulin, transferrin, selenium, sodium bicarbonate, FGF2, and TGF-β1).

The culture medium used in the present invention may contain additional components such as fatty acids or lipids, amino acids (e.g., non-essential amino acids), vitamins, cytokines, antioxidants, 2-mercaptoethanol, pyruvic acid, buffers, inorganic salts, and antibiotics.

Examples of the antibiotics that can be used include penicillin, streptomycin, and amphotericin B.

### <4. SRF Inhibitors>

The SRF inhibitors are defined as a substance that inhibits the activity of serum response factors (SRF), which are transcription factors belonging to the MADS (MCM1, Agamous, Deficiens, and SRF) box superfamily. Examples thereof include CCG-1423 (N-[2-[4(4-chlorophenyl)amino]-1-methyl-2-oxoethoxy]-3,5-bis(trifluoromethyl)-benzamide), and CCG-1423 analogs CCG-100602 (1-[3,5-bis(trifluoromethyl)benzoyl]-N-(4-chlorophenyl)-3-piperidine carboxamide), CCG-203971 (6-amino-1,4-dihydro-1,3-dimethyl-4-[4-(trifluoromethyl)phenyl]-pyrano[2,3-c]pyrazole-5-carbonitrile), CCG-222740 (Ref: Yu-Wai-Man C et al. Local delivery of novel MRTF/SRF inhibitors prevents scar tissue formation in a preclinical model of fibrosis. Sci Rep. 2017 Mar 31; 7(1): 518) and derivatives thereof, as well as antisense nucleic acids, RNA interference-induced nucleic acids (e.g., siRNA), dominant negative variants to SRF, and expression vectors thereof.

As the SRF inhibitor, one or two or more SRF inhibitors can be used.

The structural formula of N-[2-[4(4-chlorophenyl)amino]-1-methyl-2-oxoethoxy]-3,5-bis(trifluoromethyl)-benzamide described above is as follows:

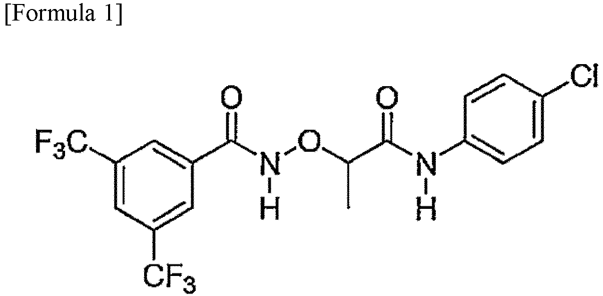

The SRF inhibitor is particularly preferably one or more selected from CCG-1423, CCG-100602, CCG-203971 and CCG-222740, most preferably CCG-1423.

### <5. ROCK Inhibitors>

The ROCK inhibitors are defined as a substance that inhibits the kinase activity of Rho kinase (ROCK, Rho-associated protein kinase). Examples thereof include Y-27632 (4-[(1R)-1-aminoethyl]-N-pyridin-4-ylcyclohexane-1-carboxamide or a salt thereof (e.g., dihydrochloride)) (see, e.g., Ishizaki et al., Mol. Pharmacol. 57, 976-983 (2000); Narumiya et al., Methods Enzymol. 325, 273-284 (2000)), H-1152 ((S)-(+)-2-methyl-1-[(4-methyl-5-isoquinolinyl)sulfonyl]-hexahydro-1H-1,4-diazepine or a salt thereof (e.g., dihydrochloride)) (see, e.g., Sasaki et al., Pharmacol. Ther. 93: 225-232 (2002)), Fasudil/HA1077 (1-(5-isoquinolinsulfonyl)homopiperazine or a salt thereof (e.g., dihydrochloride)) (see, e.g., Uenata et al., Nature 389: 990-994 (1997)), Wf-536 ((+)-(R)-4-(1-aminoethyl)-N-(4-pyridyl)benzamide monohydrochloride) (see, e.g., Nakajima et al., CancerChemother. Pharmacol. 52(4): 319-324 (2003)), Y39983 (4-[(1R)-1-Aminoethyl]-N-1H-pyrrolo[2,3-b]pyridin-4-ylbenzamide dihydrochloride), SLx-2119 (2-[3-[4-(1H-indazol-5-ylamino)-2-quinazolinyl]phenoxy]-N-(1-methylethyl)-acetamide), Azabenzimidazole-aminofurazans, DE-104, XD-4000, HMN-1152, 4-(1-aminoalkyl)-N-(4-pyridyl)cyclohexane-carboxamides, Rhostatin, BA-210, BA-207, BA-215, BA-285, BA-1037, Ki-23095, VAS-012 (see, e.g., James K. Liao et al., J Cardiovasc Pharmacol. 2007 Jul; 50(1): 17-24.) and derivatives thereof, as well as antisense nucleic acids, RNA interference-induced nucleic acids (e.g., siRNA), dominant negative variants to ROCK, and expression vectors thereof. In addition, other low-molecular-weight compounds have also been known as the ROCK inhibitors, and such compounds or derivatives thereof may be used as the ROCK inhibitors in accordance with the present invention (see, for example, US Patent Application Publication Nos. 20050209261, 20050192304, 20040014755, 20040002508, 20040002507, 20030125344, and 20030087919, and WO2003/062227, WO2003/059913, WO2003/062225, WO2002/076976, and WO2004/039796). As the ROCK inhibitor, one or two or more ROCK inhibitors can be used.

The structural formula of 4-[(1R)-1-aminoethyl]-N-pyridin-4-ylcyclohexane-1-carboxamide described above is as follows:

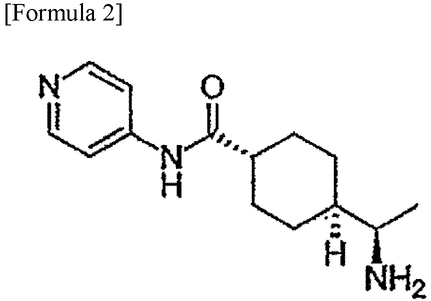

Furthermore, the structural formula of (S)-(+)-2-methyl-1-[(4-methyl-5-isoquinolinyl)sulfonyl]-hexahydro-1H-1,4-diazepine described above is as follows:

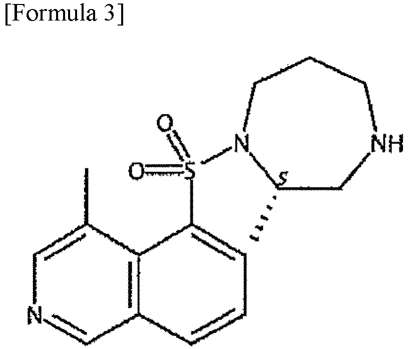

The ROCK inhibitor is particularly preferably one or more selected from Y-27632 and H-1152, and most preferably Y-27632. Y-27632 and H-1152 may be used in the form of hydrates, respectively.

### <6. Methods for Promoting Cell Aggregation>

One aspect of the present invention is a method for promoting cell aggregation, comprising a step of culturing a cell in suspension in a culture medium comprising an SRF inhibitor or an SRF inhibitor and a ROCK inhibitor (a suspension culture step).

As used herein, "promoting cell aggregation" or "cell aggregation promotion" refers to promoting aggregation of cells, thereby facilitating the formation or expansion of cell aggregates. As used herein, the "cell aggregation promoter" refers to an agent that has an effect of promoting cell aggregation.

The method of this aspect comprises the suspension culture step as an essential step, and a maintenance culture step and a collection step as optional steps. Hereinafter, each step is described.

### (Suspension Culture Step)

Specific embodiments of the step of culturing a cell in suspension in a culture medium comprising an SRF inhibitor or an SRF inhibitor and a ROCK inhibitor (a suspension culture step) are described.

Specific embodiments of the SRF inhibitor, ROCK inhibitor, culture medium, and cell are as described above.

The concentration of the SRF inhibitor in the culture medium in the suspension culture step can be adjusted appropriately according to various conditions such as type of cells, the number of cells, and type of culture medium, so that cell aggregation can be promoted. Regarding the concentration of the SRF inhibitor in the culture medium in the suspension culture step, when the SRF inhibitor is, for example, CCG-1423 (Cayman Chemical, CAS No. 285986-881, C₁₈H₁₄ClF₆N₂O₃, molecular weight = 454.8), the lower limit of the concentration of the inhibitor is not particularly limited as long as a cell aggregation promotion effect is exerted, and preferably 4.5 ng/mL or more, more preferably 45 ng/mL or more, particularly preferably 450 ng/mL or more, most preferably 1.1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, 14 µg/mL or more, 15 µg/mL or more, 16 µg/mL or more, 17 µg/mL or more, or 18 µg/mL or more. The upper limit is not particularly limited as long as the cells are not to be killed at the concentration, and preferably 4.6 mg/mL or less, more preferably 460 µg/mL or less, particularly preferably 46 µg/mL or less, and most preferably 19 µg/mL or less. The concentration of the inhibitor is preferably 4.5 ng/mL or more and 4.6 mg/mL or less. It is also particularly preferable that the concentration of the SRF inhibitor in the culture medium in the suspension culture step is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit of the concentration is preferably 10 mM or less, more preferably 1 mM or less, particularly preferably 100 µM or less, and most preferably 40 µM or less.

Furthermore, the concentration of the ROCK inhibitor in the culture medium in the suspension culture step can be adjusted according to various conditions such as type of cells, the number of cells, and type of culture medium, so that cell aggregation can be promoted appropriately. Regarding the concentration of the ROCK inhibitor in the culture medium in the suspension culture step, when the ROCK inhibitor is, for example, Y-27632 (Wako Pure Chemical Industries, Ltd., CAS No. 331752-47-7, C₁₄H₂₁N₃O·2HCl·H₂O, molecular weight = 338.27), the concentration is particularly preferably in the range of 3.3 ng/mL or more and 3.4 mg/mL or less. The lower limit of the concentration is not particularly limited as long as a cell aggregation promotion effect is exerted, and preferably 3.3 ng/mL or more, more preferably 33 ng/mL or more, particularly preferably 330 ng/mL or more, and most preferably 800 ng/mL or more, 1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, or 14 µg/mL or more. The upper limit is not particularly limited as long as the cells are not to be killed at the concentration, and preferably 3.4 mg/mL or less, more preferably 340 µg/mL or less, particularly preferably 34 µg/mL or less, and most preferably 14 µg/mL or less. It is also particularly preferable that the concentration of the ROCK inhibitor in the culture medium in the suspension culture step is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit is preferably 10 mM or less, more preferably 1 mM or less, particularly preferably 100 µM or less, and most preferably 40 µM or less.

It is preferable that the suspension culture step is a step of culturing cells in suspension under conditions where if the SRF inhibitor or the SRF inhibitor and the ROCK inhibitor of the present invention are not present in the culture medium, cell aggregates would be formed.

The cultureware used in the suspension culture step is preferably a container on which cells adhere less to an inner surface thereof. Examples of such a container include plates, the surface of which is subjected to hydrophilic treatment with a biocompatible substance. Examples of the cultureware that may be used include, but are not particularly limited to, Nunclon™ Sphera (Thermo Fisher Scientific Inc.).

Examples of the shape of the cultureware include, but are not particularly limited to, a dish, flask, well, bag, and spinner flask shape.

The suspension culture may be static culture or may be culture under conditions in which the culture medium flows (fluid culture), but preferably fluid culture. It is preferable that the fluid culture is a culture under conditions in which the culture medium flows so as to promote cell aggregation. Examples of the culture under conditions in which the culture medium flows so as to promote cell aggregation include a culture under conditions in which the culture medium flows such that cells are concentrated on a spot due to stress (centrifugal force, centripetal force) caused by a flow such as a swirling and/or rocking flow; and a culture under conditions in which the culture medium flows due to a linear back and forth movement, and particularly preferred is a culture by a swirling culture method or a rocking culture method.

The "swirling culture method" (including shaking culture method) refers to a method of culturing under conditions in which the culture medium flows such that cells are concentrated on a spot due to stress (centrifugal force, centripetal force) caused by a swirling flow. Specifically, the swirling culture method is carried out by swirling a cultureware including a culture medium containing cells in a manner to draw a closed orbit such as a circle, ellipse, flattened circle or flattened ellipse along generally a horizontal plane, or by swirling a culture medium in a cultureware with a stirrer such as a stirrer bar or stirrer blade while the cultureware is left standing. The latter may be accomplished by using, for example, a spinner flask-like cultureware with agitator blades. Such culturewares are commercially available and the commercial products may be used. In that case, the volume of the culture medium, culture solution or the like may be set as recommended by the cultureware manufacturer.

The speed of swirling in the swirling culture method is not particular limited, and the upper limit may be preferably 200 rpm or less, more preferably 150 rpm or less, still more preferably 120 rpm or less, still more preferably 115 rpm or less, still more preferably 110 rpm or less, still more preferably 105 rpm or less, furthermore preferably 100 rpm or less, still more preferably 95 rpm or less, and particularly preferably 90 rpm or less. The lower limit may be preferably 1 rpm or more, more preferably 10 rpm or more, still more preferably 50 rpm or more, still more preferably 60 rpm or more, still more preferably 70 rpm or more, still more preferably 80 rpm or more, and furthermore preferably 90 rpm or more. The swirling width during the swirling culture is not particular limited, and the lower limit may be, for example, 1 mm or more, preferably 10 mm or more, more preferably 20 mm or more, and most preferably 25 mm or more. The upper limit of the swirling width may be, for example, 200 mm or less, preferably 100 mm or less, more preferably 50 mm or less, still more preferably 30 mm or less, and most preferably 25 mm or less. The radius of rotation during the swirling culture is not particularly limited and is preferably set such that the swirling width is within the above-described range. The lower limit of the radius of rotation may be, for example, 5 mm or more, preferably 10 mm or more, and the upper limit thereof may be, for example, 100 mm or less, and preferably 50 mm or less. Setting the swirling culture condition to these ranges is preferable because it is easy to prepare cell aggregates with an appropriate size.

The "rocking culture method" refers to a method of culturing under conditions that a rocking flow is imparted to a culture medium by a linear reciprocating motion such as rocking agitation. Specifically, the rocking culture method is carried out such that a cultureware including a culture medium containing cells is rocked in a plane generally vertical to a horizontal plane. The speed of rocking is not particularly limited, and for example, when one round trip is set as one time, the rocking may be carried out with the lower limit of 2 times or more, 4 times or more, 6 times or more, 8 times or more, or 10 times or more per minute, and the upper limit of 15 times or less, 20 times or less, 25 times or less, or 50 times or less per minute. During rocking, it is preferable to impart some angle relative to the vertical surface, i.e., rocking angle, to the cultureware. The rocking angle is not particularly limited, and, for example, the lower limit may be 0° or more, 1° or more, 2° or more, 4° or more, 6° or more, or 8° or more, and the upper limit may be 10° or less, 12° or less, 15° or less, 18° or less, or 20° or less. Setting the rocking culture condition to these ranges is preferable because cell aggregates with an appropriate size can be produced.

Further, the culture may be mixed by movement in which the above rotary shaking and rocking are combined.

Culture using spinner flask-shaped cultureware in which mixing blades are placed may be carried out. During this culture, the liquid culture medium is mixed by the mixing blades. The speed of rotation and the volume of culture medium are not particularly limited. When a commercially available spinner flask-shaped cultureware is used, the volume recommended by the manufacturer may be suitably used as a volume of cell culture composition. The speed of rotation has no particular limitation and may be, for example, 10 rpm or more and 100 rpm or less.

The seeding density (i.e., the cell density at the start of suspension culture) of cells cultured in suspension in a liquid culture medium may be adjusted appropriately. The lower limit is, for example, 0.01 × 10⁵ cells/mL or more, preferably 0.1 × 10⁵ cells/mL or more, and more preferably 1 × 10⁵ cells/mL or more. The upper limit of the seeding density is, for example, 20 × 10⁵ cells/mL or less and preferably 10 × 10⁵ cells/mL or less. When the seeding density is within this range, cell aggregates with an appropriate size are likely to be formed. For example, the seeding density may be 0.1 × 10⁵ cells/mL, 0.2 × 10⁵ cells/mL, 0.3 × 10⁵ cells/mL, 0.4 × 10⁵ cells/mL, 0.5 × 10⁵ cells/mL, 0.6 × 10⁵ cells/mL, 0.7 × 10⁵ cells/mL, 0.8 × 10⁵ cells/mL, 0.9 × 10⁵ cells/mL, 1 × 10⁵ cells/mL, 1.5 × 10⁵ cells/mL, 2 × 10⁵ cells/mL, 3 × 10⁵ cells/mL, 4 × 10⁵ cells/mL, 5 × 10⁵ cells/mL, 6 × 10⁵ cells/mL, 7 × 10⁵ cells/mL, 8 × 10⁵ cells/mL, 9 × 10⁵ cells/mL, or 10 × 10⁵ cells/mL.

The volume of cell culture composition during suspension culture may be appropriately adjusted depending on the cultureware used. For example, when a 12-well plate (with a bottom area per well of 3.5 cm² in a flat view) is used, the volume may be 0.5 mL/well or more and 1.5 mL/well or less, and more preferably 1.3 mL/well. For example, when a 6-well plate (with a bottom area per well of 9.6 cm² in a flat view) is used, the volume may be 1.5 mL/well or more, preferably 2 mL/well or more, and more preferably 3 mL/well or more, and 6.0 mL/well or less, preferably 5 mL/well or less, and more preferably 4 mL/well or less. When a 125-mL Erlenmeyer flask (an Erlenmeyer flask with a volume of 125 mL) is used, for example, the volume may be 10 mL/flask or more, preferably 15 mL/flask or more, more preferably 20 mL/flask or more, still more preferably 25 mL/flask or more, still more preferably 20 mL/flask or more, still more preferably 25 mL/flask or more, and still more preferably 30 mL/flask or more. The volume may be 50 mL/flask or less, preferably 45 mL/flask or less, and more preferably 40 mL/flask or less. When a 500-mL Erlenmeyer flask (an Erlenmeyer flask with a volume of 500 mL) is used, for example, the volume may be 100 mL/flask or more, preferably 105 mL/flask or more, more preferably 110 mL/flask or more, still more preferably 115 mL/flask or more, and still more preferably 120 mL/flask or more. The volume may be 150 mL/flask or less, preferably 145 mL/flask or less, more preferably 140 mL/flask or less, still more preferably 135 mL/flask or less, still more preferably 130 mL/flask or less, and still more preferably 125 mL/flask or less. When a 1000-mL Erlenmeyer flask (an Erlenmeyer flask with a volume of 1000 mL) is used, for example, the volume may be 250 mL/flask or more, preferably 260 mL/flask or more, more preferably 270 mL/flask or more, still more preferably 280 mL/flask or more, and still more preferably 290 mL/flask or more. The volume may be 350 mL/flask or less, preferably 340 mL/flask or less, more preferably 330 mL/flask or less, still more preferably 320 mL/flask or less, and still more preferably 310 mL/flask or less. When a 2000-mL Erlenmeyer flask (an Erlenmeyer flask with a volume of 2000 mL) is used, for example, the volume may be 500 mL/flask or more, preferably 550 mL/flask or more, and more preferably 600 mL/flask or more. The volume may be 1000 mL/flask or less, preferably 900 mL/flask or less, more preferably 800 mL/flask or less, and still more preferably 700 mL/flask or less. When a 3000-mL Erlenmeyer flask (an Erlenmeyer flask with a volume of 3000 mL) is used, for example, the volume may be 1000 mL/flask or more, preferably 1100 mL/flask or more, more preferably 1200 mL/flask or more, still more preferably 1300 mL/flask or more, still more preferably 1400 mL/flask or more, and still more preferably 1500 mL/flask or more. The volume may be 2000 mL/flask or less, preferably 1900 mL/flask or less, more preferably 1800 mL/flask or less, still more preferably 1700 mL/flask or less, and still more preferably 1600 mL/flask or less. When a 2-L culture bag (a disposable culture bag with a volume of 2 L) is used, for example, the volume may be 100 mL/bag or more, preferably 200 mL/bag or more, more preferably 300 mL/bag or more, still more preferably 400 mL/bag or more, still more preferably 500 mL/bag or more, still more preferably 600 mL/bag or more, still more preferably 700 mL/bag or more, still more preferably 800 mL/bag or more, still more preferably 900 mL/bag or more, and still more preferably 1000 mL/bag or more. The volume may be 2000 mL/bag or less, preferably 1900 mL/bag or less, more preferably 1800 mL/bag or less, still more preferably 1700 mL/bag or less, still more preferably 1600 mL/bag or less, still more preferably 1500 mL/bag or less, still more preferably 1400 mL/bag or less, still more preferably 1300 mL/bag or less, still more preferably 1200 mL/bag or less, and still more preferably 1100 mL/bag or less. When a 10-L culture bag (a disposable culture bag with a volume of 10 L) is used, for example, the volume may be 500 mL/bag or more, preferably 1 L/bag or more, more preferably 2 L/bag or more, still more preferably 3 L/bag or more, still more preferably 4 L/bag or more, and still more preferably 5 L/bag or more. The volume may be 10 L/bag or less, preferably 9 L/bag or less, more preferably 8 L/bag or less, still more preferably 7 L/bag or less, and still more preferably 6 L/bag or less. When a 20-L culture bag (a disposable culture bag with a volume of 20 L) is used, for example, the volume may be 1 L/bag or more, preferably 2 L/bag or more, more preferably 3 L/bag or more, still more preferably 4 L/bag or more, still more preferably 5 L/bag or more, still more preferably 6 L/bag or more, still more preferably 7 L/bag or more, still more preferably 8 L/bag or more, still more preferably 9 L/bag or more, and still more preferably 10 L/bag or more. The volume may be 20 L/bag or less, preferably 19 L/bag or less, more preferably 18 L/bag or less, still more preferably 17 L/bag or less, still more preferably 16 L/bag or less, still more preferably 15 L/bag or less, still more preferably 14 L/bag or less, still more preferably 13 L/bag or less, still more preferably 12 L/bag or less, and still more preferably 11 L/bag or less. When a 50-L culture bag (a disposable culture bag with a volume of 50 L) is used, for example, the volume may be 1 L/bag or more, preferably 2 L/bag or more, more preferably 5 L/bag or more, still more preferably 10 L/bag or more, still more preferably 15 L/bag or more, still more preferably 20 L/bag or more, and still more preferably 25 L/bag or more. The volume may be 50 L/bag or less, preferably 45 L/bag or less, more preferably 40 L/bag or less, still more preferably 35 L/bag or less, and still more preferably 30 L/bag or less. When the volume of cell culture composition is within these ranges, cell aggregates with an appropriate size are likely to be formed.

The volume of cultureware used has no particular limitation and may be suitably selected. The area of the bottom of a portion housing a liquid culture medium may be determined in a flat view. The lower limit of the bottom area of the cultureware used may be, for example, 0.32 cm² or more, preferably 0.65 cm² or more, more preferably 0.65 cm² or more, still more preferably 1.9 cm² or more, and still more preferably 3.0 cm² or more, 3.5 cm² or more, 9.0 cm² or more, or 9.6 cm² or more. The upper limit of the bottom area of the cultureware used may be, for example, 1000 cm² or less, preferably 500 cm² or less, more preferably 300 cm² or less, still more preferably 150 cm² or less, still more preferably 75 cm² or less, still more preferably 55 cm² or less, still more preferably 25 cm² or less, still more preferably 21 cm² or less, still more preferably 9.6 cm² or less, and still more preferably 3.5 cm² or less.

Conditions such as the temperature, culture period, CO₂ level of cell suspension culture in the presence of the SRF inhibitor or the SRF inhibitor and the ROCK inhibitor are not particularly limited. The culture temperature is preferably 20°C or higher and more preferably 35°C or higher and preferably 45°C or lower and more preferably 40°C or lower. Most preferred is 37°C. The culture period is preferably 0.5 hour or longer and more preferably 12 hours or longer and preferably 7 days or shorter, more preferably 72 hours or shorter, still more preferably 48 hours or shorter, and most preferably 24 hours or shorter. The CO₂ level during the culture is preferably 4% or higher and more preferably 4.5% or higher and preferably 10% or lower and more preferably 5.5% or lower. Most preferred is 5%. Furthermore, in the methods for promoting cell aggregation of the present invention, a passage procedure may be accompanied during the suspension culture step. When the culture conditions are within these ranges, cell aggregates with an appropriate size are likely to be formed. Furthermore, the culture medium can be exchanged in an appropriate frequency. The frequency of the culture medium exchange may vary depending on the cell species to be cultured, but, for example, may be one or more times every 5 days, one or more times every 4 days, one or more times every 3 days, one or more times every 2 days, one or more times a day. The culture medium exchange may be carried out by collecting cells in the same manner as in the collection step described below, then adding a fresh culture medium, subsequently gently dispersing cell aggregates; and then culturing again.

In the suspension culture step, to what extent the number of cells is increased and to which the state of cells is to be met may be determined appropriately depending on the type of cells to be cultured, the purpose of cell aggregation, the type of culture medium, and the culture conditions.

It is preferable that the cells used in the suspension culture step are cells cultured by a maintenance culture step in advance, then collected by a collection step, and single-cellularized as needed. The maintenance culture step, collection step, and single-cellularization are as described below.

After the suspension culture step, the culture solution is discarded by a common procedure and the cells are collected. At this time, the cells are preferably collected as single cells by a detachment or dispersion treatment. Specific methods thereof are described in detail in the collection step described below. The collected cells may be directly, or after being washed with a buffer (including a PBS buffer), saline, or culture medium (preferably a culture medium used in the next step or a basal medium) as needed, subjected to the next step.

### (Maintenance Culture Step)

A "maintenance culture step" is a step of culturing a cell population before a suspension culture step, or a cell aggregate obtained after a suspension culture step or after a subsequent collection step to proliferate the cells while remaining undifferentiated. The maintenance culture may be adherent culture in which cells are cultured while adhered to a culture substrate such as a container or a support, or may be suspension culture in which cells are cultured in suspension in a culture medium.

In the maintenance culture step, cells of interest may be cultured by known animal cell culture methods in the art. The culture in the maintenance culture step may be adhesion culture or suspension culture.

Specific embodiments of the culture medium and cells used in the maintenance culture step are as described above.

The cultureware, seeding density of cells, and culture conditions used in the maintenance culture step are as described above with respect to the suspension culture step.

The flow state of the culture medium in the maintenance culture step is not limited. The maintenance culture may be static culture or fluid culture.

The "static culture" refers to culturing cells while standing the culture medium in a cultureware. In adhesion culture, this static culture is typically employed.

The "fluid culture" refers to culturing cells under conditions in which the culture medium is flowed. Specific embodiments of the fluid culture are as described above with respect to the suspension culture step.

In the maintenance culture step, to what extent the number of cells is increased and to which the state of cells is to be met may be determined appropriately depending on the type of cells to be cultured, the purpose of cell aggregation, the type of culture medium, and the culture conditions.

A suitable aspect of the maintenance culture step is a maintenance culture step that further cultures cell aggregates formed by a suspension culture step in the presence of an SRF inhibitor or an SRF inhibitor and a ROCK inhibitor. The method of culture in the maintenance culture step in this aspect is not particularly limited, and examples thereof include a step of culturing cell aggregates in suspension in a culture medium free of SRF inhibitors and ROCK inhibitors. As the culture medium used for this maintenance culture, the same culture medium as described above except that SRF inhibitors and ROCK inhibitors are not contained can be used. As the conditions for the maintenance culture, the same conditions as those in the suspension culture step can be used. In the maintenance culture step in this aspect, it is preferable that the culture medium is exchanged in an appropriate frequency. The frequency of the medium change may vary depending on a type of the cells. The frequency of medium change operation may be preferably once or more per 5 days, more preferably once or more per 4 days, still more preferably once or more per 3 days, still more preferably once or more per 2 days, and most preferably once or more per day. This frequency of the culture medium exchange is particularly suitable when cell aggregates of stem cells are cultured. Methods of the culture medium exchange are not particularly limited, and a preferable method may include: collecting all the volume of the cell aggregate-containing cell culture composition into a centrifuge tube; subjecting the tube to centrifugation or a standing state for about 5 minutes; removing the supernatant from precipitated cell aggregates; then adding a fresh culture medium; gently dispersing the cell aggregates, and then returning the cell aggregate-dispersed culture medium to a cultureware such as a plate, so that the cell aggregates can be cultured continuously. The culture period of the maintenance culture step in this aspect is not particularly limited, and preferably 3 days or more and 7 days or less. By further culturing the cell aggregates in suspension in a culture medium free of SRF inhibitors and ROCK inhibitors in the conditions described above, cell aggregates with an appropriate size can be obtained.

After the maintenance culture step, the culture solution is discarded by a common procedure and the cells are collected. At this time, the cells are preferably collected as single cells by a detachment or dispersion treatment. Specific methods thereof are described in detail in the collection step described below. The collected cells may be directly, or after being washed with a buffer (including a PBS buffer), saline, or culture medium (preferably a culture medium used in the next step or a basal medium) as needed, subjected to the next step.

### (Collection Step)

The "collection step" is a step of collecting cultured cells from culture solution after a maintenance culture step or a suspension culture step, and is an optional step in the method of the present invention.

As used herein, "collection (of cells)" refers to separating cells from a culture solution to obtain the cells. The collection method of cells may follow a common procedure used in cell culture methods in the art, and is not particularly limited. The cell culture methods can generally be classified into suspension culture methods and adhesion culture methods. Hereinafter, the collection method of cells after each culture method will be described.

### (Collection Method After Suspension Culture Method)

When cells are cultured in a suspension culture method, the cells are present in a suspension state in the culture solution. Thus, collection of cells can be accomplished by removing liquid components of the supernatant in the static state or by centrifugation. Furthermore, filters, hollow filament separation membranes or the like can be selected as collection methods of cells.

In the case of removing liquid components in the static state, the container containing the culture solution may be left in the static state for about 5 minutes, and the supernatant may be removed to leave the deposited cells or cell aggregates. Centrifugation may also be performed at a rotational speed and processing time at which cells are not damaged by centrifugal forces. For example, the lower limit of the rotational speed is not particularly limited as long as the cells can be deposited, and may be, for example, 500 rpm or more, 800 rpm or more, or 1000 rpm or more. While, the upper limit is not limited as long as the cells do not suffer or are not vulnerable to damage by centrifugal forces at the rotational speed, and may be, for example, 1400 rpm or less, 1500 rpm or less, or 1600 rpm or less. The lower limit of the processing time is not particularly limited as long as the cells can be deposited at the rotational speed, and may be, for example, 30 seconds or more, 1 minute or more, 3 minutes or more, or 5 minutes or more. The upper limit is not limited as long as the cells do not or hardly suffer from damage by the rotation, and may be, for example, 30 seconds or less, 6 minutes or less, 8 minutes or less, or 10 minutes or less. The collected cells can be washed as needed. Methods for washing are not limited. For example, the same methods as the washing method after the maintenance culture step described above can be used. A buffer (including a PBS buffer), saline, or culture medium (preferably, basal medium) may be used as a washing solution.

### (Collection Method After Adhesion Culture)

When cells are cultured in an adhesion culture method, many cultured cells are present while adhered to an external matrix such as a cultureware and a culture support. Thus, to remove the culture solution from the cultureware, the cultureware may be gently tilted after the culture to drain liquid components. Since the cells adhered to the external matrix remain in the cultureware, the cells and the culture solution can be readily separated.

Cell surfaces adhered to the external matrix can then be washed as needed. A buffer (including a PBS buffer), saline, or culture medium (preferably, basal medium) may be used as a washing solution. However, the washing solution is not limited thereto. The washing solution after washing may be removed in the same manner as the culture solution. This washing step may be repeated multiple times.

The cell population adhered to the external matrix is then detached from the external matrix. The detachment method may be performed in a manner known in the art. Typically, scraping, detaching agents containing proteolytic enzymes as active ingredients, chelating agents such as EDTA, or mixtures of detaching agents and chelating agents, or the like are used.

Scraping is a method for stripping cells attached to an external matrix by mechanical means such as scrapers. However, since cells are vulnerable to damage by mechanical procedures, when the collected cells are subjected to further culture, it is preferable to employ a detachment method which chemically destroys or degrades the scaffold portion of cells bound to an external matrix and releases the adhesion between the cells and the external matrix.

In the detachment method, a detaching agent and/or a chelating agent are used. The detaching agent is not limited, and examples thereof include trypsin, collagenase, pronase, hyaluronidase, elastase, as well as commercially available Accutase (registered trade mark), TrypLE™ Express Enzyme (Life Technologies Japan Ltd.), TrypLE™ Select Enzyme (Life Technologies Japan Ltd.), "Dispase" (registered trade mark). The concentration and processing time of each detaching agent may be set in the range of those in common procedures for cell detachment or dispersion. For example, when the detaching agent is trypsin, the lower limit of the concentration in the solution is not particularly limited as long as the cells can be detached at the concentration, and may be, for example, 0.01% or more, 0.02% or more, 0.03% or more, 0.04% or more, 0.05% or more, 0.08% or more, or 0.10% or more. The upper limit of the concentration in the solution is not particularly limited as long as cells themselves are not affected with lysis or the like by the action of trypsin at the concentration, and may be, for example, 0.15% or less, 0.20% or less, 0.25% or less, or 0.30% or less. The processing time also depends on the concentration of trypsin, but the lower limit is not particularly limited as long as the cells can be sufficiently detached from the external matrix by the action of trypsin at the time, and may be, for example, 1 minute or more, 2 minutes or more, 3 minutes or more, 4 minutes or more, or 5 minutes or more. The upper limit of the processing time is not particularly limited as long as cells themselves are not affected with lysis or the like by the action of trypsin at the time, and may be, for example, 8 minutes or less, 10 minutes or less, 12 minutes or less, 15 minutes or less, 18 minutes or less, or 20 minutes or less. Other detaching agents and chelating agents can be used generally in the same manner as described above. When commercially available detaching agents are used, the concentrations and processing times described in the attached protocol can be employed.

The cells detached from the external matrix are separated from the supernatant containing detaching agents by centrifugation. The centrifugal conditions may be the same as those in the "Collection Method After Suspension Culture Method" described above. The collected cells can be washed as needed. The washing method may also be carried out in the same manner as those in the "Collection Method After Suspension Culture Method" described above.

The cells obtained after this step may partially include cell assemblies such as monolayer cell fragments and cell aggregates. The collected cells can be single-cellularized as needed.

### (Single-cellularization)

As used herein, "single-cellularization" refers to dispersing cell assemblies such as monolayer cell fragments and cell aggregates in which multiple cells are adhered or aggregated each other to make a state of single free cells.

Single-cellularization can be performed by increasing the concentration of detaching agents and/or chelating agents and/or by extending the processing time with detaching agents and/or chelating agents used in the above-described detachment method. For example, when the detaching agent is trypsin, the lower limit of the concentration in the solution is not particularly limited as long as cell assemblies can be dispersed at the concentration, and may be, for example, 0.15% or more, 0.18% or more, 0.20% or more, or 0.24% or more. The upper limit of the concentration in the solution is not particularly limited as long as the cells themselves are not affected with lysis or the like at the concentration, and may be 0.25% or less, 0.28% or less, or 0.30% or less. The processing time also depends on the concentration of trypsin, but the lower limit is not particularly limited, as long as cell assemblies can be sufficiently dispersed by the action of trypsin at the time, and may be, for example, 5 minutes or more, 8 minutes or more, 10 minutes or more, 12 minutes or more, or 15 minutes or more. The upper limit of the processing time is not particularly limited as long as the cells themselves are not affected with lysis or the like by the action of trypsin at the time, and may be, for example, 18 minutes or less, 20 minutes or less, 22 minutes or less, 25 minutes or less, 28 minutes or less, or 30 minutes or less. When commercially available detaching agents are used, the agent may be used at the concentration at which the cells can be dispersed to be a single state as described in the attached protocol. Single-cellularization can be facilitated by physically lightly treating cells after treating with the detaching agent and/or chelating agent. This physical treatment is not limited, and examples thereof include a method of pipetting cells together with the solution multiple times. Additionally, cells may be passed through a strainer or mesh as needed.

Single-cellularized cells can be collected by removing supernatants containing detaching agents by standing or centrifugation. The collected cells may be washed as needed. Conditions for centrifugation and methods for washing can be carried out in the same manner as in the "Collection Method After Suspension Culture Method" described above.

### <7. Cell Aggregation Promoters>

Another aspect of the present invention is a cell aggregation promoter for use in suspension culture of cells, comprising an SRF inhibitor or an SRF inhibitor and a ROCK inhibitor.

The cell aggregation promoter of the present invention can be used for moderately promoting cell aggregation in the suspension culture system, and forming cell aggregates with a substantially uniform size. In suspension culture of stem cells using the cell aggregation promoter of the present invention, the stem cells can remain undifferentiated.

The form of the cell aggregation promoter according to the present invention is not particularly limited, and may be an SRF inhibitor, or an SRF inhibitor and a ROCK inhibitor alone, or a composition of an SRF inhibitor, or an SRF inhibitor and a ROCK inhibitor in combination with other components. The form of the composition is not particularly limited. The composition may be, for example, a form of a culture medium used for suspension culture or may be a form of an additive composition mixed when a culture medium is prepared.

A preferred embodiment of the cell aggregation promoter according to the present invention is a culture medium or a buffer such as a phosphate buffer comprising an SRF inhibitor, or an SRF inhibitor and a ROCK inhibitor. Examples of the concentrations of the SRF inhibitor and the ROCK inhibitor in the culture medium include the concentrations of the SRF inhibitor and the ROCK inhibitor in a culture medium described for suspension culture in the column <6. Methods for Promoting Cell Aggregation>.

Another preferred embodiment of the cell aggregation promoter according to the present invention is a liquid or solid composition comprising an SRF inhibitor or an SRF inhibitor and a ROCK inhibitor in a liquid or solid medium. The liquid or solid composition is an additive which is added when a culture medium for suspension culture is prepared. It is preferable that the cell aggregation promoter of this embodiment is prepared such that the final concentrations of the SRF inhibitor and the ROCK inhibitor in the culture medium to be prepared are the concentrations of the SRF inhibitor and the ROCK inhibitor in the culture medium described for suspension culture in the column <6. Methods for Promoting Cell Aggregation>. The concentration of the SRF inhibitor in the cell aggregation promoter of this embodiment is not particularly limited, and preferably 1 or more, more preferably 2 or more, still more preferably 10 or more, still more preferably 100 or more, still more preferably 1000 or more, and still more preferably 10000 or more times the above-mentioned concentration of the SRF inhibitor specified as a preferable concentration in the culture medium during suspension culture. Specifically, for example, when the SRF inhibitor is CCG-1423 (Cayman Chemical, CAS No. 285986-881, C₁₈H₁₄ClF₆N₂O₃, molecular weight = 454.8), it is also particularly preferable that the concentration of the SRF inhibitor is in the range of 9.0 µg/mL or more and 10.0 mg/mL or less. The lower limit of the concentration is not particularly limited as long as the cell aggregation promotion effect is exerted, and preferably 9.0 µg/mL or more, 10.0 µg/mL or more, 20.0 µg/mL or more, 30.0 µg/mL or more, 40.0 µg/mL or more, 50.0 µg/mL or more, 60.0 µg/mL or more, 70.0 µg/mL or more, 80.0 µg/mL or more, 90.0 µg/mL or more, or 100.0 µg/mL or more. The upper limit is not particularly limited as long as the cells are not to be killed at the concentration, and preferably 10.0 mg/mL or less, 9.0 mg/mL or less, 8.0 mg/mL or less, 7.0 mg/mL or less, 6.0 mg/mL or less, 5.0 mg/mL or less, 4.0 mg/mL or less, 3.0 mg/mL or less, 2.0 mg/mL or less, 1.0 mg/mL or less, 900.0 µg/mL or less, 800.0 µg/mL or less, 700.0 µg/mL or less, 600.0 µg/mL or less, or 500.0 µg/mL or less. It is also particularly preferable that the concentration of the SRF inhibitor is in a range of 20 µM or more and 20 mM or less. The lower limit of the concentration of the SRF inhibitor is, for example, 20 µM or more, 200 µM or more, 0.05 mM or more, 0.1 mM or more, 0.2 mM or more, 0.3 mM or more, 0.4 mM or more, 0.5 mM or more, 0.6 mM or more, 0.7 mM or more, 0.8 mM or more, 0.9 mM or more, 1 mM or more, 2 mM or more, 3 mM or more, 4 mM or more, 5 mM or more, 6 mM or more, 7 mM or more, 8 mM or more, 9 mM or more, 10 mM or more, 11 mM or more, 12 mM or more, 13 mM or more, 14 mM or more, or 15 mM or more. The upper limit thereof is, for example, 20 mM or less. Furthermore, the concentration of the ROCK inhibitor in the cell aggregation promoter of this embodiment is not particularly limited, and, preferably 1 or more, more preferably 2 or more, still more preferably 10 or more, still more preferably 100 or more, still more preferably 1000 or more, and still more preferably 10000 or more times the above-mentioned concentration of the ROCK inhibitor specified as a preferable concentration in the culture medium during suspension culture. Specifically, for example, when the ROCK inhibitor is Y-27632 (Wako Pure Chemical Industries, Ltd., CAS No. 331752-47-7, C₁₄H₂₁N₃O·2HCl·H₂O, molecular weight = 338.27), it is particularly preferable that the concentration thereof is in the range of 6.7 µg/mL or more and 14.0 mg/mL or less. For example, the concentration thereof may be 6.7 mg/mL. The lower limit of the concentration is not particularly limited as long as the cell aggregation promotion effect is exerted, and preferably 6.7 µg/mL or more, 7.0 µg/mL or more, 8.0 µg/mL or more, 9.0 µg/mL or more, 10.0 µg/mL or more, 20.0 µg/mL or more, 30.0 µg/mL or more, 40.0 µg/mL or more, 50.0 µg/mL or more, 60.0 µg/mL or more, 70.0 µg/mL or more, 80.0 µg/mL or more, 90.0 µg/mL or more, or 100.0 µg/mL or more. The upper limit is not particularly limited as long as the cells are not to be killed at the concentration, and preferably 14.0 mg/mL or less, 13.0 mg/mL or less, 12.0 mg/mL or less, 11.0 mg/mL or less, 10.0 mg/mL or less, 9.0 mg/mL or less, 8.0 mg/mL or less, 7.0 mg/mL or less, 6.0 mg/mL or less, 5.0 mg/mL or less, 4.0 mg/mL or less, 3.0 mg/mL or less, 2.0 mg/mL or less, 1.0 mg/mL or less, 900.0 µg/mL or less, 800.0 µg/mL or less, 700.0 µg/mL or less, 600.0 µg/mL or less, or 500.0 µg/mL or less. It is also particularly preferable that the concentration of the ROCK inhibitor ranges from 20 µM or more and 40 mM or less. The lower limit of the concentration of the ROCK inhibitor is, for example, 20 µM or more, 200 µM or more, 0.5 mM or more, 0.6 mM or more, 0.7 mM or more, 0.8 mM or more, 0.9 mM or more, 1 mM or more, 2 mM or more, 3 mM or more, 4 mM or more, 5 mM or more, 6 mM or more, 7 mM or more, 8 mM or more, 9 mM or more, 10 mM or more, 11 mM or more, 12 mM or more, 13 mM or more, 14 mM or more, 15 mM or more, 16 mM or more, 17 mM or more, 18 mM or more, 19 mM or more, or 20 mM or more. The upper limit thereof is, for example, 40 mM or less, or 30 mM or less.

The cell aggregation promoter may comprise, in addition to the SRF inhibitor or the SRF inhibitor and the ROCK inhibitor, a solvent and/or an excipient. Examples of the solvent include water, buffers (including PBS), saline, and organic solvents (DMSO, DMF, xylene, lower alcohol). Examples of the excipient include an antibiotic, buffer, thickener, colorant, stabilizer, surfactant, emulsifier, preservative, preserving agent, and antioxidant. Examples of the antibiotics that can be used include, but are not particularly limited to, penicillin, streptomycin, and amphotericin B. Examples of the buffer include a phosphate buffer, tris-hydrochloric acid buffer, and glycine buffer. Examples of the thickener include gelatin and polysaccharides. Examples of the colorant include Phenol Red. Examples of the stabilizer include albumin, dextran, methyl cellulose, and gelatin. Examples of the surfactant include cholesterol, an alkyl glycoside, alkyl polyglycoside, alkyl monoglyceride ether, glucoside, maltoside, neopentyl glycol series, polyoxyethylene glycol series, thioglucoside, thiomaltoside, peptide, saponin, phospholipid, sorbitan fatty acid ester, and fatty acid diethanolamide. Examples of the emulsifier include a glycerin fatty acid ester, sorbitan fatty acid ester, propylene glycol fatty acid ester, and sucrose fatty acid ester. Examples of the preservative include aminoethyl sulfonic acid, benzoic acid, sodium benzoate, ethanol, sodium edetate, agar, dl-camphor, citric acid, sodium citrate, salicylic acid, sodium salicylate, phenyl salicylate, dibutylhydroxy toluene, sorbic acid, potassium sorbate, nitrogen, dehydro acetic acid, sodium dehydroacetate, 2-naphthol, white soft sugar, honey, paraoxy isobutyl benzoate, paraoxy isopropyl benzoate, paraoxy ethyl benzoate, paraoxy butyl benzoate, paraoxy propyl benzoate, paraoxy methyl benzoate, 1-menthol, and eucalyptus oil. Examples of the preserving agent include benzoic acid, sodium benzoate, ethanol, sodium edetate, dried sodium sulfite, citric acid, glycerin, salicylic acid, sodium salicylate, dibutylhydroxy toluene, D-sorbitol, sorbic acid, potassium sorbate, sodium dehydroacetate, paraoxy isobutyl benzoate, paraoxy isopropyl benzoate, paraoxy ethyl benzoate, paraoxy butyl benzoate, paraoxy propyl benzoate, paraoxy methyl benzoate, propylene glycol, and phosphoric acid. Examples of the antioxidant include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, super oxide dismutase (SOD), glutathione peroxidase, glutathione-S-transferase, glutathione reductase, catalase, ascorbic acid peroxidase, and mixtures thereof.

The cell aggregation promoter may contain a growth factor, preferably one or more growth factors of FGF2 and TGF-β1.

### <8. Method for Producing Cell Aggregates, and Cell Aggregates Produced Thereby>

Another aspect of the present invention is a method for producing a cell aggregate, comprising a step of culturing a cell in suspension in a culture medium comprising an SRF inhibitor or an SRF inhibitor and a ROCK inhibitor.

According to this method, cell aggregates with appropriate size can be produced in high yields. In particular, when the cells are stem cells, cell aggregates with appropriate size in which the stem cells remain undifferentiated can be produced in high yields.

Specific embodiments of the SRF inhibitor, ROCK inhibitor, culture medium, and cell are as described above.

Regarding the concentration of the SRF inhibitor in the culture medium in the step described above, when the SRF inhibitor is, for example, CCG-1423 (Cayman Chemical, CAS No. 285986-881; C₁₈H₁₄ClF₆N₂O₃; molecular weight = 454.8), the lower limit of the concentration of the inhibitor is not particularly limited as long as the cell aggregation promotion effect is exerted, and preferably 4.5 ng/mL or more, more preferably 45 ng/mL or more, particularly preferably 450 ng/mL or more, and most preferably 1.1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, 14 µg/mL or more, 15 µg/mL or more, 16 µg/mL or more, 17 µg/mL or more, 18 µg/mL or more, or 19 µg/mL or more. The upper limit is not particularly limited as long as the cells are not to be killed at the concentration, and preferably 4.6 mg/mL or less, more preferably 460 µg/mL or less, and particularly preferably 46 µg/mL or less, 40 µg/mL or less, 30 µg/mL or less, or 20 µg/mL or less. The concentration of the inhibitor is preferably 4.5 ng/mL or more and 4.6 mg/mL or less. It is also particularly preferable that the concentration of the SRF inhibitor in the culture medium in the step described above is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit is preferably 10 mM or less, more preferably 1 mM or less, particularly preferably 100 µM or less, and most preferably 40 µM or less.

Furthermore, regarding the concentration of the ROCK inhibitor in the culture medium in the step described above, when the ROCK inhibitor is, for example, Y-27632 (Wako Pure Chemical Industries, Ltd., CAS No. 331752-47-7, C₁₄H₂₁N₃O·2HCl·H₂O, molecular weight = 338.27), the concentration is particularly preferably in the range of 3.3 ng/mL or more and 3.4 mg/mL or less. The lower limit of the concentration is not particularly limited as long as the cell aggregation promotion effect is exerted, and preferably 3.3 ng/mL or more, more preferably 33 ng/mL or more, particularly preferably 330 ng/mL or more, and most preferably 800 ng/mL or more, 1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, or 14 µg/mL or more. The upper limit is not particularly limited as long as the cells are not to be killed at the concentration, and preferably 3.4 mg/mL or less, more preferably 340 µg/mL or less, particularly preferably 34 µg/mL or less, and most preferably 14 µg/mL or less. It is also particularly preferable that the concentration of the ROCK inhibitor in the culture medium in the step is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit is preferably 10 mM or less, more preferably 1 mM or less, particularly preferably 100 µM or less, and most preferably 40 µM or less.

Specific embodiments of the step described above are similar to the specific embodiments of the "Step of culturing cells in suspension in a culture medium comprising an SRF inhibitor or an SRF inhibitor and a ROCK inhibitor" in the method for promoting cell aggregation described in the column <6. Methods for Promoting Cell Aggregation>.

Another aspect of the present invention is a cell aggregate obtained by the method for producing cell aggregate described above.

The cell aggregate according to this aspect of the present invention has an appropriate size and high viable cell ratio. Furthermore, when the cells are stem cells, the cells constituting the cell aggregate remain undifferentiated.

The cell aggregate according to this aspect of the present invention preferably has the features described in the column <2. Cell Aggregates>.

The method for producing a cell aggregate of the present invention can also appropriately comprise an optional step, in addition to a suspension culture step of culturing cells in suspension in a culture medium comprising an SRF inhibitor or an SRF inhibitor and a ROCK inhibitor. Examples of the optional step include a maintenance culture step and a collection step of cell aggregates. Furthermore, the suspension culture may include a passage procedure. Suitable embodiments of the maintenance culture step and the collection step are similar to the maintenance culture step and the collection step described in the column <6. Methods for Promoting Cell Aggregation>.

### <9. Cell Culture Composition>

Another aspect of the present invention is a cell culture composition comprising a cell, a culture medium, and an SRF inhibitor, or an SRF inhibitor and a ROCK inhibitor.

The cell culture composition according to this aspect of the present invention can be used to produce cell aggregates in high yields. In particular, when the cells are stem cells, the cell culture composition can be used to produce cell aggregates with appropriate size, in which the stem cells remain undifferentiated, in high yields.

Furthermore, the cell culture composition according to this aspect of the present invention may comprise cells in a form of cell aggregates.

Specific embodiments of the SRF inhibitor, ROCK inhibitor, culture medium, cell, and cell aggregate are as described above.

Regarding the concentration of the SRF inhibitor in the cell culture composition according to this aspect of the present invention, when the SRF inhibitor is, for example, CCG-1423 (Cayman Chemical, CAS No. 285986-881; C₁₈H₁₄ClF₆N₂O₃; molecular weight = 454.8), the lower limit of the concentration of the inhibitor is not particularly limited as long as the cell aggregation promotion effect is exerted, and preferably 4.5 ng/mL or more, more preferably 45 ng/mL or more, particularly preferably 450 ng/mL or more, most preferably 1.1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, 14 µg/mL or more, 15 µg/mL or more, 16 µg/mL or more, 17 µg/mL or more, or 18 µg/mL or more. The upper limit is not particularly limited as long as the cells are not to be killed at the concentration, and preferably 4.6 mg/mL or less, more preferably 460 µg/mL or less, particularly preferably 46 µg/mL or less, and most preferably 19 µg/mL or less. The concentration of the inhibitor is preferably 4.5 ng/mL or more and 4.6 mg/mL or less. It is also particularly preferable that the concentration of the SRF inhibitor in the cell culture composition according to this aspect of the present invention is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit is preferably 10 mM or less, more preferably 1 mM or less, particularly preferably 100 µM or less, and most preferably 40 µM or less.

Furthermore, regarding the concentration of the ROCK inhibitor in the cell culture composition according to this aspect of the present invention, when the ROCK inhibitor is, for example, Y-27632 (Wako Pure Chemical Industries, Ltd., CAS No. 331752-47-7, C₁₄H₂₁N₃O·2HCl·H₂O, molecular weight = 338.27), the concentration is particularly preferably in the range of 3.3 ng/mL or more and 3.4 mg/mL or less. The lower limit of the concentration is not particularly limited as long as the cell aggregation promotion effect is exerted, and preferably 3.3 ng/mL or more, more preferably 33 ng/mL or more, particularly preferably 330 ng/mL or more, and most preferably 800 ng/mL or more, 1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, or 14 µg/mL or more. The upper limit is not particularly limited as long as the cells are not to be killed at the concentration, and preferably 3.4 mg/mL or less, more preferably 340 µg/mL or less, particularly preferably 34 µg/mL or less, and most preferably 14 µg/mL or less. It is also particularly preferable that the concentration of the ROCK inhibitor in the cell culture composition according to this aspect of the present invention is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit is preferably 10 mM or less, more preferably 1 mM or less, particularly preferably 100 µM or less, and most preferably 40 µM or less.

Examples of the step of producing cell aggregates from the cell culture composition include a step of culturing cells in suspension in the cell culture composition described above. Specific embodiments of this step are similar to the specific embodiments of the "step of culturing a cell in suspension in a culture medium comprising an SRF inhibitor or an SRF inhibitor and a ROCK inhibitor" in the method for promoting cell aggregation described in the column <6. Methods for Promoting Cell Aggregation>.

The cell culture composition may be prepared by adding an SRF inhibitor or an SRF inhibitor and a ROCK inhibitor to a culture medium, then adding cells to the culture medium, or by mixing cells with a culture medium, then adding an SRF inhibitor or an SRF inhibitor and a ROCK inhibitor to the mixture. Preferably, the cell culture composition is prepared by adding an SRF inhibitor or an SRF inhibitor and a ROCK inhibitor to a culture medium, then adding cells to the culture medium. A stabilizer can also be added when adding the SRF inhibitor or the SRF inhibitor and the ROCK inhibitor to the culture medium. The stabilizer is not particularly limited as long as it is a substance that can contribute to, for example, stabilization of the SRF inhibitor or the SRF inhibitor and the ROCK inhibitor in a liquid culture medium, maintenance of its activity, and prevention of adsorption to the cultureware, and examples thereof include protein such as albumin, an emulsifier, a surfactant, an amphiphilic substance, or a polysaccharide compound such as heparin.

The cell culture composition may be prepared by freezing and storing a culture medium comprising an SRF inhibitor or an SRF inhibitor and a ROCK inhibitor (which may further comprise the stabilizer), then thawing and adding cells to the culture medium.

### <10. Cell Culture Medium>

Another aspect of the present invention is a cell culture medium comprising a culture medium, and an SRF inhibitor or an SRF inhibitor and a ROCK inhibitor.

The cell culture medium according to this aspect of the present invention can be used as a culture medium for producing cell aggregates from cells by suspension culture in high yields. In particular, when the cells are stem cells, the cell culture medium can be used to produce cell aggregates with appropriate size, in which the stem cells remain undifferentiated, in high yields.

Specific embodiments of the SRF inhibitor, ROCK inhibitor, culture medium, and cell are as described above.

Regarding the concentration of the SRF inhibitor in the cell culture medium according to this aspect of the present invention, when the SRF inhibitor is, for example, CCG-1423 (Cayman Chemical, CAS No. 285986-881; C₁₈H₁₄ClF₆N₂O₃; molecular weight = 454.8), the lower limit of the concentration of the inhibitor is not particularly limited as long as the cell aggregation promotion effect is exerted, and preferably 4.5 ng/mL or more, and more preferably 45 ng/mL or more, particularly preferably 450 ng/mL or more, most preferably 1.1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, 14 µg/mL or more, 15 µg/mL or more, 16 µg/mL or more, 17 µg/mL or more, or 18 µg/mL or more. The upper limit is not particularly limited as long as the cells are not to be killed at the concentration, and preferably 4.6 mg/mL or less, more preferably 460 µg/mL or less, particularly preferably 46 µg/mL or less, and most preferably 19 µg/mL or less. The concentration of the inhibitor is preferably 4.5 ng/mL or more and 4.6 mg/mL or less. It is also particularly preferable that the concentration of the SRF inhibitor in the cell culture medium according to this aspect of the present invention is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit is preferably 10 mM or less, more preferably 1 mM or less, particularly preferably 100 µM or less, most preferably 40 µM or less.

Furthermore, regarding the concentration of the ROCK inhibitor in the cell culture medium according to this aspect of the present invention, when the ROCK inhibitor is, for example, Y-27632 (Wako Pure Chemical Industries, Ltd., CAS No. 331752-47-7, C₁₄H₂₁N₃O·2HCl·H₂O, molecular weight = 338.27), the concentration is particularly preferably in the range of 3.3 ng/mL or more and 3.4 mg/mL or less. The lower limit of the concentration is not particularly limited as long as the cell aggregation promotion effect is exerted, and preferably 3.3 ng/mL or more, more preferably 33 ng/mL or more, particularly preferably 330 ng/mL or more, most preferably 800 ng/mL or more, 1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, 13 µg/mL or more, or 14 µg/mL or more. The upper limit is not particularly limited as long as the cells are not to be killed at the concentration, and preferably 3.4 mg/mL or less, more preferably 340 µg/mL or less, particularly preferably 34 µg/mL or less, and most preferably 14 µg/mL or less. It is also particularly preferable that the concentration of the ROCK inhibitor in the cell culture medium according to this aspect of the present invention is in the range of 10 nM or more and 10 mM or less. The lower limit of the concentration is preferably 10 nM or more, more preferably 100 nM or more, particularly preferably 1 µM or more, and most preferably 2.5 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, or 39 µM or more. The upper limit is preferably 10 mM or less, more preferably 1 mM or less, particularly preferably 100 µM or less, and most preferably 40 µM or less.

Examples of the step of producing cell aggregates from the cell culture medium described above include a step of culturing cells in suspension in the cell culture medium. Specific embodiments of this step are similar to the specific embodiments of the "step of culturing a cell in suspension in a culture medium comprising an SRF inhibitor or an SRF inhibitor and a ROCK inhibitor" in the method for promoting cell aggregation described in the column <6. Methods for Promoting Cell Aggregation>.

The cell culture medium can be frozen and stored until use and thawed upon use.

### Examples

### <Example 1: Maintenance Culture of Human iPS Cells>

TkDN4-M cell lines (Institute of Medical Science, The University of Tokyo) were used as human iPS cells. Human iPS cells were seeded on cell culture dishes coated with Vitronectin (Thermo Fisher Scientific Co., Ltd.) and subjected to a maintenance culture using Essential 8™ (Thermo Fisher Scientific Co., Ltd.) as the culture medium. Accutase (Thermo Fisher Scientific Co., Ltd.) was used as a cell detachment agent during passage. In addition, when the cells were seeded, Y-27632 (Wako Pure Chemical Industries, Ltd.) at a concentration of 10 µM was added to a culture medium. The culture medium was changed every day. For experiments, human iPS cells (the number of passage was 50 or less) were used.

### <Example 2. Confirmation of Aggregation Promotion Effect by Cell Aggregation Assay>

The cell aggregation promotion effect by adding an SRF inhibitor was investigated.

### (Protocol)

Human iPS cells that had been cultured using the protocol of Example 1 were treated with Accutase for 3 to 5 minutes and were detached and dispersed to single cells. The resulting cells were suspended in Essential 8™ culture medium containing a final concentration of 5 mg/mL of BSA (Wako Pure Chemical Industries, Ltd.) and a final concentration of 2.5 µM of Y-27632 (Wako Pure Chemical Industries, Ltd.), and a portion thereof was stained with trypan blue and the number of cells was counted. The cell suspension was prepared so as to contain 2 × 10⁵ cells per ml. Separately, the cell aggregation promoter was adjusted so that the final concentration of CCG-1423 (Cayman, 10010350) was 4.55 mg/mL (10 mM), then the adjusted cell aggregation promoter was added to the cell suspension so that the final concentration of CCG-1423 was 10 µM. Then the cells were seeded at a ratio of 1.3 mL/well in a 12-well plate for suspension culture (Sumitomo Bakelite Co., Ltd.). The cell-seeded plate was subjected to a swirling culture on a rotary shaker (OPTIMA, Inc.) at a speed of 90 rpm along the horizontal plane to draw a circle with a swirling width (diameter) of 25 mm and cells were cultured in suspension under a condition at 5% CO₂ and 37°C. A control test was conducted using the cell suspension prepared in the same way as described above except that CCG-1423 was not added.

At the next day after the start of culture (Day 1 of culture), images were obtained by phase contrast microscopy, and the number of dead cells was calculated with Cytotoxicity LDH Assay Kit-WST (Peer Chemistry Research Institute, Inc.).

### (Results)

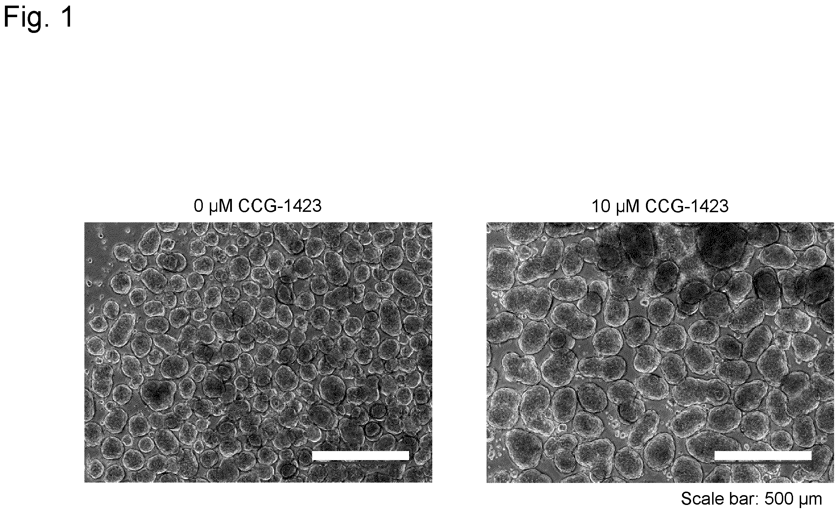

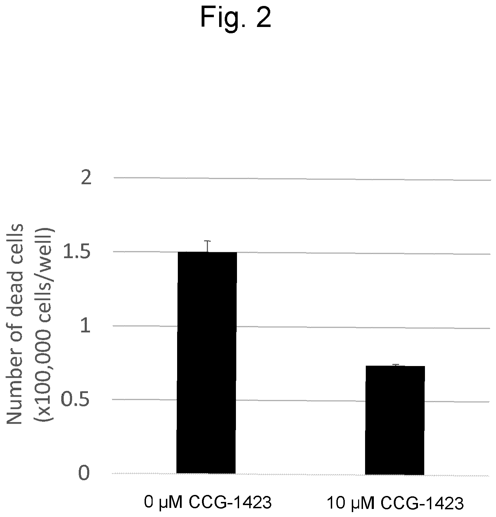

Figure 1 shows micrographs after the above suspension culture (Day 1 of culture). As a result of the observations, relatively large aggregates were formed, and aggregation was promoted under CCG-1423 added condition compared to the control test (0 µM CCG-1423). The result of examination of the number of dead cells showed that the number of dead cells was reduced under CCG-1423 added condition compared to the control test (0 µM CCG-1423).(Figure 2).

### <Example 3: Effect of Presence of CCG-1423 on Cell Proliferation Potential and Undifferentiated State After Formation of Aggregate>

Suspension culture of human iPS cells was performed, and the glucose consumption, the cell yield, and the percentage of cells positive for undifferentiation markers were determined to analyze the effect of CCG-1423 on cells.

### (Protocol)

A cell suspension was prepared in the same manner as in Example 2, and separately, the cell aggregation promoter was adjusted so that the final concentration of CCG-1423 (same as above) was 4.55 mg/mL (10 mM), then the adjusted cell aggregation promoter was added to the cell suspension so that the final concentration of CCG-1423 was 10 µM, and the cells were seeded at a ratio of 4 mL/well in a 6-well plate for suspension culture (Sumitomo Bakelite Co., Ltd.). The cell-seeded plate was subjected to a swirling culture on a rotary shaker (OPTIMA, Inc.) at a speed of 90 rpm along the horizontal plane to draw a circle with a swirling width (diameter) of 25 mm and cells were cultured in suspension under a condition at 5% CO₂ and 37°C. After the next day of culture (Day 1 of culture), the culture medium was exchanged daily for a fresh culture medium (Essential 8™ culture medium containing BSA (Wako Pure Chemical Industries, Ltd.) at a final concentration of 5 mg/mL), and the culture continued until 5 days after the start of culture. A control test was conducted by culturing in the same way except for using CCG-1423 and Y-27632 free culture media. Images were obtained by phase contrast microscopy every day during culture. While 208 cell aggregates in images obtained at Day 1 of culture being observed and compared using a micrograph scale, the width (referred to as "ϕ") of the widest portion of each cell aggregate was measured, distribution thereof was examined, and average ± standard deviation was calculated. The concentration of glucose contained in the culture supernatant collected at the time of culture medium exchange was measured with biosensor BF-5iD (Prince Measuring Equipment Co., Ltd.) to calculate glucose consumption.

In addition, at Day 5 of culture, cell aggregates were collected, dispersed with Accutase, and then suspended in Essential 8™ culture medium containing 5 mg/mL BSA. A portion of this cell suspension was stained with trypan blue and the number of cells was counted. After the above cell suspensions were centrifuged at 300 g for 3 minutes, the supernatant was then removed, and the cells were washed with PBS (phosphate buffered saline). Next, the cells were fixed with 4% paraformaldehyde (Wako Pure Chemical Industries, Ltd.) at room temperature for 20 minutes, then washed 3 times with PBS. After cells were resuspended with 300 µL of PBS, 3 mL of cold methanol was added while stirring with voltex, and permeabilized at -20°C overnight or more. After 3 washes with 3% FBS (fetal bovine serum)/PBS, cells were resuspended with 3% FBS (fetal bovine serum)/PBS and blocked at room temperature for 30 minutes to 1 hour. Subsequently, the cells were stained with fluorescently labeled anti-SOX2 antibodies (Cat. No. 656110, BioLegend, Inc.) and fluorescently labeled anti-OCT4 antibodies (Cat. No. 653703, BioLegend, Inc.) and fluorescently labeled anti-Nanog antibodies (Cat. No. 674010, BioLegend, Inc.) at 4°C for 30 minutes to 1 hour. After washed once with 3% FBS (fetal bovine serum)/PBS, the cells were made to pass through a cell strainer. The resulting cells were analyzed on FACSVerse. A control test was conducted using cells that were treated in the same way except for reacting, instead of the above three antibodies (fluorescently labeled anti-SOX2 antibody, fluorescently labeled anti-OCT4 antibody, fluorescently labeled anti-Nanog antibody), with three fluorescently labeled isotype control antibodies (Cat. No. 400129, Cat. No. 400314, Cat. No. 400136; BioLegend, Inc.) corresponding to each of the above three antibodies.

Cell yields were also measured at Day 5 of culture. The following procedure was used to measure the cell yields. Specifically, the cell aggregates that had been formed were treated with Accutase for 5 to 10 minutes, pipetted using a blue tip to monodisperse cells, and stained with trypan blue. After that, the number of cells was counted using a hemocytometer to determine the cell yield.

### (Results)

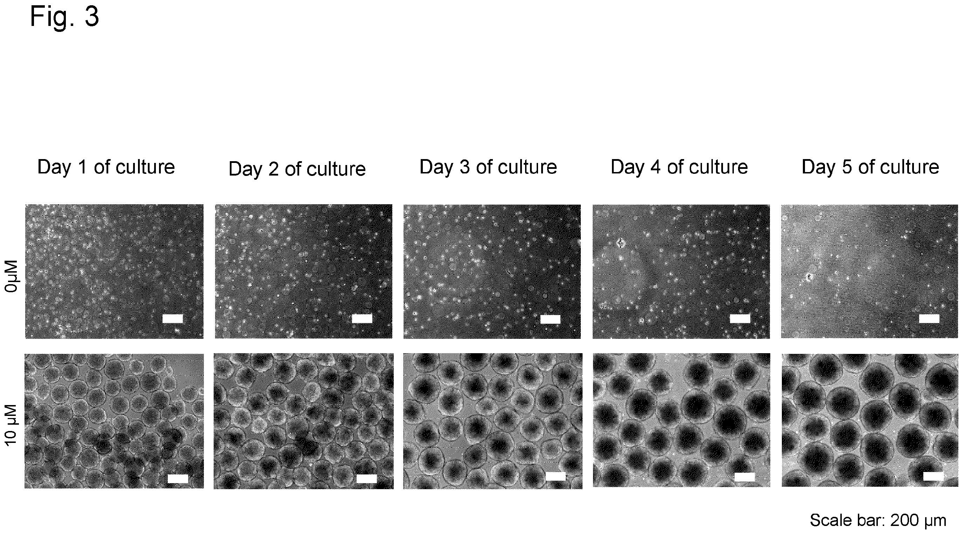

Figure 3 is micrographs obtained at Day 1 to Day 5 of culture. Under CCG-1423 added condition, cell aggregates were formed after seeding to Day 1 of culture, and by continuing the culture, cells were gradually proliferated and cell aggregates were expanded.

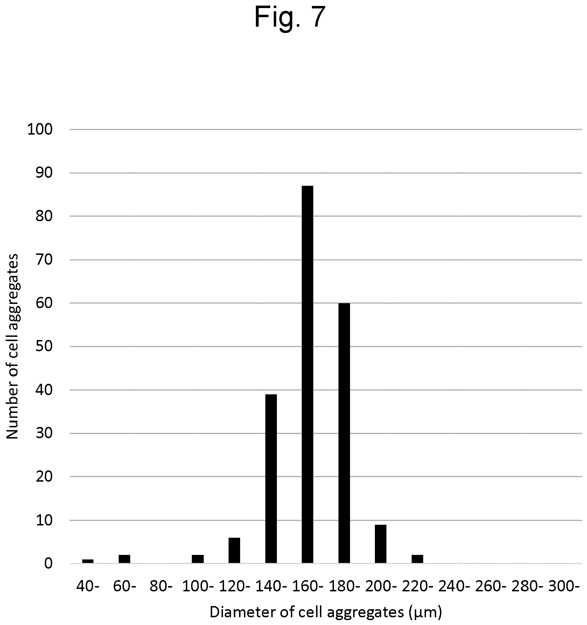

Figure 7 is a distribution of cell aggregate size (diameter) at Day 1 of culture under CCG-1423 added condition. Table 1 below also shows the number and percentage of cell aggregates by size at Day 1 of culture under CCG-1423 added condition.

The result of calculation of an average ± standard deviation of cell aggregate size (diameter) at Day 1 of culture under CCG-1423 added condition was 171.3 ± 22.8 µm.

Furthermore, at Day 1 of culture under CCG-1423 added condition, the percentage of cell aggregates whose cell aggregate size (diameter) was 40 µm or more and 300 µm or less was 100% to the total cell aggregate count. Among them, the percentage of cell aggregates whose cell aggregate size (diameter) was 60 µm or more and 300 µm or less was 99.5%, the percentage of cell aggregates whose cell aggregate size (diameter) was 80 µm or more and 300 µm or less was 98.5%, and the percentage of cell aggregates whose cell aggregate size (diameter) was 100 µm or more and 300 µm or less was 98.5%.

### [Table 1]

**Table 1. Number and percentage of cell aggregates by size**

| Size range (µm) | 40- | 60- | 80- | 100- | 120- | 140- | 160- | 180- | 200- | 220- | 240- | 260- | 280- | 300- |
|---|---|---|---|---|---|---|---|---|---|---|---|---|---|---|
| Number | 1 | 2 | 0 | 2 | 6 | 39 | 87 | 60 | 9 | 2 | 0 | 0 | 0 | 0 |
| Percentage | 0.5% | 1.0% | 0.0% | 1.0% | 2.9% | 18.8% | 41.8% | 28.8% | 4.3% | 1.0% | 0.0% | 0.0% | 0.0% | 0.0% |

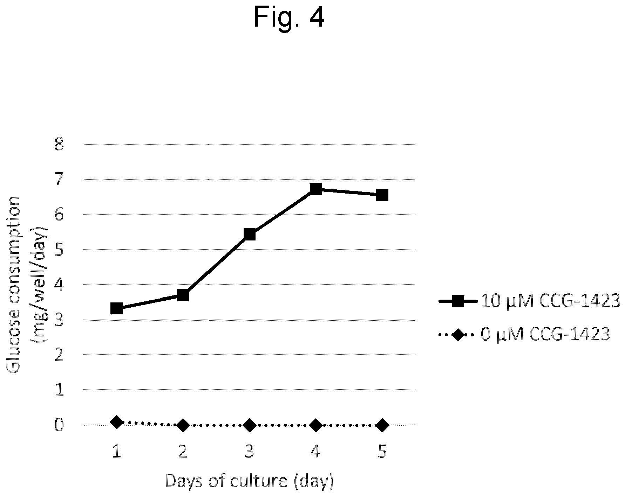

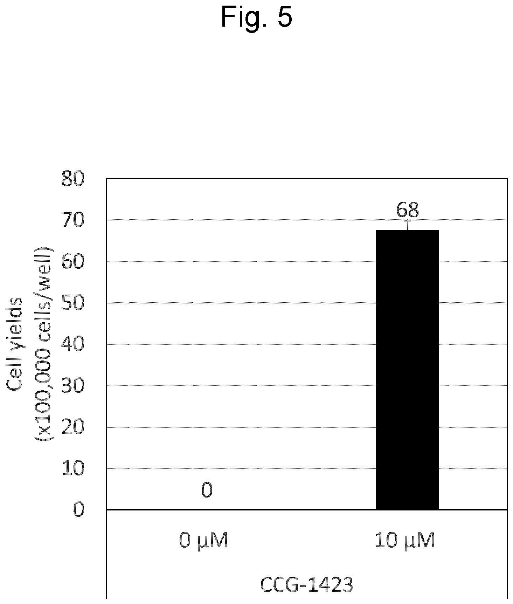

Glucose consumption is shown in Figure 4, and cell yields at Day 5 of culture are shown in Figure 5, respectively. Under CCG-1423 added condition, glucose consumption increased daily, suggesting that cells were proliferating. It was revealed that the number of seeded cells (8 x 10⁵ cells/well) had proliferated approximately 8.5-fold at Day 5 of culture.

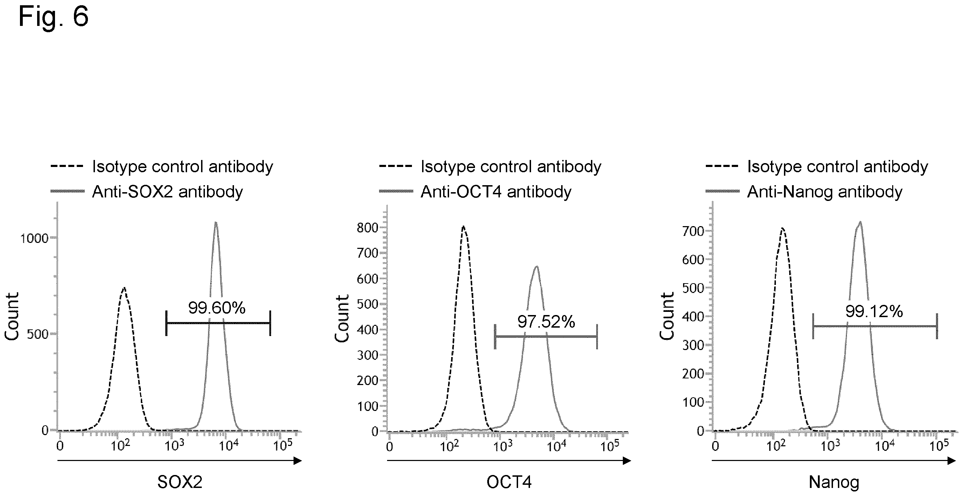

Figure 6 shows the results of measuring the percentage of cells positive for undifferentiation markers. In cells obtained by producing cell aggregates in a culture medium comprising CCG-1423 followed by proliferation in suspension culture, the percentages of cells positive for SOX2, OCT4 and Nanog, which were markers, were found to be 99% or more, 97% or more, and 99% or more, respectively. This result verified that the human iPS cell aggregates formed by the addition of CCG-1423 remained undifferentiated.

All the publications, patents, and patent applications cited herein are incorporated herein by reference in its entirety.

## Claims

1. A cell aggregation promoter for use in suspension culture of cells, comprising an SRF inhibitor.

2. The cell aggregation promoter according to claim 1, wherein a concentration of the SRF inhibitor is 9.0 µg/mL or more and 10.0 mg/mL or less.

3. The cell aggregation promoter according to claim 1 or 2, further comprising a ROCK inhibitor.

4. The cell aggregation promoter according to any one of claims 1 to 3, wherein the SRF inhibitor is CCG-1423.

5. The cell aggregation promoter according to any one of claims 1 to 4, wherein the cells are stem cells.

6. A method for producing cell aggregates, comprising a step of culturing cells in suspension in a culture medium comprising an SRF inhibitor.

7. The method according to claim 6, wherein a concentration of the SRF inhibitor in the culture medium is 4.5 ng/mL or more and 4.6 mg/mL or less.

8. The method according to claim 6 or 7, wherein the culture medium further comprises a ROCK inhibitor.

9. The method according to any one of claims 6 to 8, wherein the SRF inhibitor is CCG-1423.

10. The method according to any one of claims 6 to 9, wherein the cells are stem cells.

11. A cell aggregate obtained by the method according to any one of claims 6 to 10.

12. A cell culture composition comprising cells, a culture medium, and an SRF inhibitor.

13. The cell culture composition according to claim 12, wherein a concentration of the SRF inhibitor is 4.5 ng/mL or more and 4.6 mg/mL or less.

14. The cell culture composition according to claim 12 or 13, further comprising a ROCK inhibitor.

15. The cell culture composition according to any one of claims 12 to 14, wherein the SRF inhibitor is CCG-1423.

16. The cell culture composition according to any one of claims 12 to 15, wherein the cells are stem cells.

17. The cell culture composition according to any one of claims 12 to 16, wherein the cells are in a form of cell aggregates.
